# EUROPÄISCHE PATENTSCHRIFT

(11) **EP 2 405 802 B1**
(45) Veröffentlichungstag und Bekanntmachung des Hinweises auf die Patenterteilung: **05.12.2012**
(21) Anmeldenummer: 10708974.0
(22) Anmeldetag: 10.03.2010
(51) Int. Cl.: A61B 5/00, A61B 5/145

(54) **PROBENAHMEVORRICHTUNG UND -VERFAHREN**
SAMPLING DEVICE AND SAMPLING METHOD
DISPOSITIF ET PROCÉDÉ D'ÉCHANTILLONNAGE

(30) Priorität: 10.03.2009 DE 102009001455; 03.03.2010 WO PCT/EP2010/052727
(43) Veröffentlichungstag der Anmeldung: 18.01.2012
(73) Patentinhaber: Trace Analytics GmbH, 38106 Braunschweig (DE)
(72) Erfinder: KÜNNECKE, Wolfgang, 38116 Braunschweig (DE)
(74) Vertreter: Eisenführ, Speiser & Partner
(86) Internationale Anmeldenummer: PCT/EP2010/053052
(87) Internationale Veröffentlichungsnummer: WO 2010/103051

(56) Entgegenhaltungen:
- WO-A2-2008/059050
- GB-A- 2 100 859
- US-A- 4 265 249

## Beschreibung

Die vorliegende Erfindung betrifft eine Probenahmevorrichtung, ein Probenahmesystem und ein Probenahmeverfahren, insbesondere ein Analyseverfahren zur Anwendung an einem Lebewesen.

Die Erfindung betrifft das Gebiet der Bestimmung der Anwesenheit und/oder der Konzentration eines Analyten in einem zu beprobenden Medium. Derartige Bestimmungsaufgaben treten insbesondere bei der kontinuierlichen und diskontinuierlichen Überwachung von Stoffwechselprozessen in einem Lebewesen wie beispielsweise einem Menschen, bei biotechnischen Kultivierungen oder bei anderen technischen Prozessen auf. Gewünscht ist jeweils eine möglichst zeitnahe und möglichst genaue Bestimmung des Analyten, also seiner Anwesenheit und/oder seiner Konzentration im zu beprobenden Medium, ohne dabei das zu untersuchende Medium und die darin gegebenenfalls ablaufenden Prozesse zu stören oder für die Analyse erhebliche Mengen des Mediums zu verbrauchen.

Die Erfindung wird nachfolgend hauptsächlich im Hinblick auf Anwendungen am menschlichen oder tierischen Körper beschrieben, insbesondere im Hinblick auf medizinische Analyseaufgaben. Die Erfindung ist jedoch nicht auf dieses Gebiet beschränkt. Soweit also nachfolgend Ausdrücke aus dem medizinischen Bereich wie beispielsweise Kanäle oder Katheter verwendet werden, sind damit als weitere Ausführungsformen auch entsprechende Gegenstände der chemischen und biotechnologischen Industrie gemeint, beispielsweise Rohre und Schläuche.

Die Bestimmung eines Analyten in einem Medium erfolgt gewöhnlich durch unmittelbares Inkontaktbringen eines für den jeweiligen Analysten geeigneten Sensors mit dem zu untersuchenden Medium selbst oder einer Probe des Mediums, die aus einem Mediumhauptkörper entnommen wurde. So wird beispielsweise in der medizinischen Analytik versucht, miniaturisierte Sensoren unmittelbar in einem Patienten oder dabei vorzugsweise in seinem Blutstrom anzuordnen. Der jeweilige Sensor soll dann kontinuierlich oder zu einem vorgewählten Zeitpunkt eine oder mehrere zugeordnete Analyten bestimmen, also seine/ihre Anwesenheit und/oder Konzentration im Medium feststellen und das Ergebnis dieser Feststellung in geeigneter Form zugänglich machen, beispielsweise als elektrisches Signal oder als optisches Signal oder als Funksignal. Dabei ist problematisch, dass ein im Inneren eines Patienten angeordneter Sensor, beispielsweise ein im Blutstrom befindlicher oder implantierter Sensor, nur schlecht kalibriert werden kann, da hierfür ein Kalibriermedium in dem Patienten so eingebracht werden muss, dass es am Sensorort jedes Medium des Körpers so deutlich verdrängt, dass eine Verfälschung der Kalibrierung durch Einflüsse von außerhalb des Kalibriermediums weitgehend oder vollständig ausgeschlossen werden können. Dies ist sehr aufwendig und wegen des damit verbundenen Risiko eines Eingriffs in den Patienten in manchen Fällen nicht möglich.

In der medizinischen Analytik wird deshalb auch versucht, durch Anbringen eines Probenahmezugangs am Patienten kontinuierlich oder zu einem vorgewählten Zeitpunkt eine Probe eines Mediums des Patienten zu gewinnen, beispielsweise eine Blutprobe. Diese Probe wird dann zu einem Sensor geleitet und anschließend verworfen oder dem Patienten wieder zugeführt. Die Wiederzuführung der Probe ist insbesondere unter dem Gesichtspunkt empfehlenswert, den Patienten durch eine wiederholte Probenahme nicht unnötig zu belasten, indem er gleichsam ausgeblutet würde. Bei den beschriebenen Analyseverfahren durch Gewinnen einer Probe befindet sich der Sensor also nicht mehr unmittelbar im Hauptkörper des zu analysierenden Mediums, sondern im Probenahmezugang, beispielsweise in einem Katheter oder einer Kanüle, und analysiert eine in den Probenahmezugang einströmende oder eingeströmte oder eingesogene Probe des Mediums. Ebenso kann der Sensor auch in noch größerem Abstand vom Mediumhauptkörper angeordnet sein, beispielsweise an einem vom Mediumhauptkörper abgewandten Ende des Probenahmezugangs. Und schließlich kann der Sensor auch von einer fluidleitenden Verbindung zum Mediumhauptkörper völlig getrennt sein, beispielsweise indem eine isolierte Blutprobe einem externen Sensor zugeführt wird, beispielsweise einem Teststreifen.

Bei nicht im Inneren des Mediumhauptkörpers angeordneten Sensoren ist zu beachten, dass das zu beprobende Medium sich bereits durch die Entnahme vom Mediumhauptkörper in seiner Beschaffenheit ändern kann. So kann beispielsweise eine Blutprobe, die rasch durch eine Kanüle angesaugt wird, starken Scherkräften unterworfen sein, was zu Schädigungsartefakten führen kann. Ebenfalls ist zu besorgen, dass insbesondere Blutproben gerinnen. Ein solches Gerinnen kann beispielsweise auch im Inneren einer Kanüle oder eines Venenverweilkatheters erfolgen. Dies ist nicht nur zum Sicherstellen aussagekräftiger Sensorsignale zu vermeiden, sondern auch, um das Einbringen von Thromben in den Patienten zu vermeiden.

Bei medizinischen Durchfluss-Analyseverfahren ist es häufig notwendig, die zu vermessende Probe für eine bestimmte Kontaktzeit in einer Messzelle stehen zu lassen, da Strömungen und Pulsationen beispielsweise durch Herzschlag oder Blutdruckschwankungen Sensoren beeinflussen können.

Es ist deshalb versucht worden, den Sensor und das zu beprobende Medium voneinander durch Zwischenschalten eines Transportmediums zu entkoppeln. Dabei wird zunächst das Transportmedium mit dem zu analysierenden Medium in Kontakt gebracht, um das Transportmedium mit dem/den Analyten zu beladen. Anschließend wird die analytbeladene Probe des Transportmediums zum Sensor transportiert und dort vermessen. Vorteilhaft an solchen Verfahren ist, dass durch Zwischenschalten eines Transportmediums die am Sensor herrschenden Bedingungen wie beispielsweise Druck und Temperatur vorwählbar sind und genauer gesteuert werden können als in dem zu analysierenden Medium selbst. Bei geeigneter Verfahrensführung kann zudem vermieden werden, dem Mediumhauptkörper ein überhaupt ein merkliches Volumen zu entnehmen.

Ein Beispiel für solche Verfahren sind Dialyseverfahren. Dabei wird eine Sonde in das zu untersuchende Medium eingeführt und gegebenenfalls für längere Zeit implantiert, wobei die Sonde ein Transportmedium enthält, das über ein mit einer Dialyse- oder Gasdiffusionsmembran bedecktes Messfenster in Stoffaustauschverbindung mit dem zu untersuchenden Medium gebracht wird. Die Sonde wird mit dem Transportmedium kontinuierlich oder pulsweise gespült. Durch die Membran und das Messfenster tritt der Analyt in das Transportmedium einer in Analyt-Strömungsrichtung hinter der Membran liegenden Analytaufnahmekammer ein und wird aus der Sonde durch einen Sondenausgang aus dem Bereich des zu untersuchenden Mediums, insbesondere einem Bioreaktor oder einem menschlichen oder tierischen Körper, heraustransportiert. Beispiele solcher Sonden und zugehöriger Probenahmeverfahren sind beschrieben in der DE 44 26 694, US 3 640 269, US 4 008 717, US 4 221 567, US 4 694 832, US 6 632 315, US 6 811 542, US 6 852 500, US 7 162 290, US 2008-97288, WO 99/45982 A2, WO 01/06928 A1, WO 2004/032735 A2, WO 2001/010483 A1 und WO 2008/059050 A2.

Gerade dann, wenn die Sonde im Körper eines Lebewesens, insbesondere eines Menschen oder eines Tieres, angeordnet ist, kann die Konzentration der aus der Sonde heraustransportierten Analyten im Transportmedium unabhängig von der Konzentration im Körper des Lebewesens stark schwanken. Die Erfinder vermuten, dass durch Bewegungen des Lebewesens die das Messfenster verschließende Membran in Kontakt mit Gewebe oder anderem festen Material des Lebewesens gerät und dabei ganz oder teilweise so bedeckt wird, dass der Übertritt des Analyten in die Analytaufnahmekammer behindert wird. Ferner haben die Erfinder festgestellt, dass die Konzentration des Analyten im Transportmedium stark abhängt von der Fließrate des zu analysierenden Mediums, insbesondere von der Fließrate von Blut.

Ein weiterer Nachteil ist, dass herkömmliche Mikrodialysesonden mit steigender Länge aufgrund ihres geringen Außendurchmessers unhandlich flexibel werden. Beispielsweise ist zum Einführen in bzw. durch das Innenlumen eines Zentralvenenkatheters eine Mikrodialysesonden-Länge von rund 30 cm notwendig. Derartige lange Mikrodialysesonden sind nur schwer in einen Katheter gegen den ausfließenden Blutstrom einführbar, was die Bedienung der Mikrodialysesonde erschwert und Beschädigungen der Sonde beispielsweise durch Abknicken begünstigt. Selbst vergleichsweise kurze Mikrodialysesonden von beispielsweise 10 cm Länge zum Einsatz in einen konventionellen Venenverweilkatheter (Braunüle) lassen sich nur mit viel Geschick einfädeln. Ferner kommt erschwerend hinzu, dass sich innerhalb von Kanülen und Kathetern an Aufweitungen und Verbindern Kanten befinden, an denen die Spitze einer Mikrodialysesonde hängen bleiben und gegebenenfalls abgeknickt oder anderweitig beschädigt werden kann.

Ein weiterer Nachteil ist, dass die Form des blutgefüllten Innenraums eines Katheters oder einer Kanüle bei eingesetzter Mikrodialysesonde unregelmäßiger wird oder es gar zur Bildung von Toträumen kommen kann, in denen die Durchströmung mit Blut nicht optimal ist. In solchen Fällen vergrößert sich die Neigung zur Verstopfung, gegebenenfalls zur Ansammlung von Luftblasen und insgesamt die Verfälschung des Sensorsignals sowie die Gefährdung des Patienten.

Es war deshalb die Aufgabe der vorliegenden Erfindung, den oben beschriebenen Nachteilen durch Angabe einer Probenahmevorrichtung, eines Probenahmesystems und eines Probenahme- und/oder Analyseverfahrens abzuhelfen. Die Probenahmevorrichtung, das Probenahmesystem und die Verfahren sollten möglichst vielseitig und möglichst im klinischen Alltag anwendbar sein. Die Probenahmevorrichtung und das Probenahmesystem sollten leicht zu sterilisieren und steril zu halten sein. Die Gefahr von Verstopfungen und Luftblasenansammlungen sollte verringert werden. Die Probenahmevorrichtung und das Probenahmesystem sollten möglichst preiswert herstellbar sein. Sie sollten eine Probenahme auch ohne Austausch der einzelnen Probenahmezugänge über einen Zeitraum von bis zu 8 Tagen erlauben, und dabei insbesondere die Schwankungsbreite und das Ausmaß unvermeidlicher Probenahmeartefakte bei Anwendung am Menschen und insbesondere am Blutkreislauf des Menschen (z.B. Hämolyse) gering halten.

Diese und weitere Aufgaben werden erfindungsgemäß gelöst durch eine Probenahmevorrichtung, ein Probenahmesystem, ein Probenahmeverfahren und ein Analyseverfahren wie nachfolgend beschrieben.

Erfindungsgemäß angegeben wird deshalb eine Probenahmevorrichtung für einen Probenahmezugang, umfassend
- eine Analytaufnahmekammer zum Aufnehmen eines Analyten in einem Transportmedium, verbunden mit einem Zuleitungskanal und einem Ableitungskanal zum Transportieren von Transportmedium in die bzw. von der Analytaufnahmekammer,
- eine als innere Ausnehmung ausgebildete Probenkammer mit einem Probeneinlass zum Aufnehmen einer gegebenenfalls analythaltigen Probe und
- ein die innere Ausnehmung umgebendes Trennmedium zum Durchtretenlassen des Analyten aus der Probenkammer in die Analytaufnahmekammer.

Die erfindungsgemäße Probenahmevorrichtung ist auf einen Probenahmezugang bezogen. Ein Probenahmezugang ist ein Körper, der den Zugang zu einem zu beprobenden Medium vermittelt. Besonders bevorzugt ist die Probenahmevorrichtung bezogen auf einen medizinischen Probenahmezugang, vorzugsweise eine Hohlnadel oder Kanüle, einem Katheter, einem Port, einem Sauger, einer Drainage, einem Reservoir und/oder einem Konnektor. Die erfindungsgemäße Probenahmevorrichtung ist vorzugsweise insbesondere angepasst zum Durchführen, Einbringen in oder anderweitigem Anbringen an einem solchen Probenahmezugang, insbesondere einem medizinischen Probenahmezugang und zweckmäßigerweise einem medizinischen Probenahmezugang der beschriebenen Art. Durch diese Anpassung ist die erfindungsgemäße Probenahmevorrichtung auf vorteilhafte Weise eingerichtet, mit einem zu untersuchenden Medium und insbesondere einer Körperflüssigkeit eines Menschen und/oder Tieres zum Gewinnen einer Probe in Kontakt zu treten. Ebenso kann die erfindungsgemäße Probenahmevorrichtung angepasst sein an einen Probenahmezugang eines chemischen Reaktors oder Bioreaktors, insbesondere an einen Reaktorstutzen wie beispielsweise Stutzen für Messwertgeber. Der Fachmann wird zur Auslegung solcher Stutzen und zum entsprechenden Anpassen einer erfindungsgemäßen Probenahmevorrichtung beispielsweise heranziehen: Chmiel, Bioprozesstechnik, Einführung in die Bioverfahrenstechnik, 1. Auflage, 1999, Kapitel 8.11 und 8.12. Die erfindungsgemäße Probenahmevorrichtung ist also sehr vielseitig einsetzbar.

Die Probenahmevorrichtung besitzt eine Probenahmekammer, die als eine innere Ausnehmung mit einem Probeneinlass zum Aufnehmen einer gegebenenfalls analythaltigen Probe ausgebildet ist. Diese innere Ausnehmung ist von einem Trennmedium umgeben, wobei das Trennmedium dem Durchtritt eines Analyten aus der Probenkammer in eine angrenzende Analytaufnahmekammer ermöglicht. Anders als bei herkömmlichen Mikrodialysesonden befindet sich das Trennmedium also nicht auf der Außenseite, sondern im Inneren der erfindungsgemäßen Probenahmevorrichtung. Dies ermöglicht es auf vorteilhaft einfache und gleichzeitig sichere Weise, eine mechanische Einwirkung auf das Trennmedium außer durch die zu analysierende Probe weitgehend oder vollständig zu vermeiden. Es kommt also vorteilhafterweise nur noch höchst selten und in besonders gelagerten Fällen dazu, dass das Trennmedium durch einen anderen Körper als das zu beprobende Medium bedeckt wird. Verschlüsse von Teilen des Trennmediums, wie sie bei einem Anbringen des Trennmediums auf der Außenseite der Probenahmevorrichtung beispielsweise durch eine Blutgefäßwand, aber auch durch eine Kanülen- oder Kathetenrvand erfolgen können, sind mit der erfindungsgemäßen Probenahmevorrichtung praktisch ausgeschlossen. Dies vergrößert auf vorteilhaft einfache Weise die Reproduzierbarkeit und Sicherheit der Probenahme auch bei längerem Verweilen der erfindungsgemäßen Probenahmevorrichtung in einem zu beprobenden Medium.

Durch das Vorsehen eines analytdurchlässigen Trennmediums ist es auf vorteilhaft einfache Weise möglich, das Transportmedium von einer Vielzahl solcher Stoffe freizuhalten, die das Bestimmen eines gewählten Analyten in einem Sensor behindern können. Im Ergebnis wird durch das Trennmedium ein Teil des Analyten von der Probe abgetrennt und in die Analytaufnahmekammer und das darin befindliche Transportmedium durchtreten gelassen. Es kommt also in der Analytaufnahmekammer zu einer Analytanreicherung im Transportmedium. Der Fachmann wählt die Größe der Probenahmekammer, das Material, die Form und die Dicke des Trennmediums zweckmäßigerweise in Ansehung des jeweiligen Analyten und des jeweiligen Mediums. Dabei orientiert sich der Fachmann insbesondere an für den jeweiligen Analyten und das jeweilige zu beprobende Medium üblichen Trennmedien, beispielsweise Dialyse- und Gasdiffusionsmembranen.

Erfindungsgemäß bevorzugt und im Folgenden mit dem Begriff "erfindungsgemäße Probenahmevorrichtung" jeweils auch gemeint ist eine solche erfindungsgemäße Probenahmevorrichtung, bei der die Analytaufnahmekammer die Probenkammer umgibt. Die Analytaufnahmekammer bildet somit im Querschnitt gleichsam einen Ring, der einen inneren Kreis, also die Probenkammer, umschließt. Bei einer nicht erfindungsgemäß angeordneten Analytaufnahmekammer, die nur an einer Stelle an die Probenkammer angrenzt, kann hingegen Analyt nur an dieser einen Stelle von der Probenkammer in die Analytaufnahmekammer übertreten, nicht aber beispielsweise auf der dieser Stelle im Querschnitt gegenüberliegenden Seite der Probenkammer. Die eine Probenkammer umgebende Analytaufnahmekammer kann also erfindungsgemäß beispielsweise auch ausgebildet sein als ein die Probenkammer umgebender Kanal, beispielsweise indem der Kanal um die Probenkammer schraubenförmig oder mäandrierend herumläuft.

Auf diese Weise wird eine vorteilhaft große Oberfläche, durch die ein Analyt aus der Probenkammer in die Analytaufnahmekammer durchtreten kann, relativ zum Volumen der Analytaufnahmekammer erzeugt. Dies ermöglicht im Vergleich mit einer Probenahmevorrichtung, deren Analytaufnahmekammer die Probenkammer wie oben beschrieben nicht umgibt, eine vorteilhaft hohe Durchtrittsrate des Analyten in das Transportmedium; die benötigte Zeit zum Anreichern einer vorgewählten Menge an Analyten im Transportmedium gegenüber kleineren Flächen kann vorteilhaft verringert werden, so das die Messfrequenz erhöht werden kann und zudem die Messsicherheit erhöht wird, indem es unwahrscheinlicher gemacht wird, dass eventuell in der Probe vorhandene Partikel einen signifikanten Teil der Fläche für den Analytdurchtritt blockieren können.

Besonders bevorzugt und im Folgenden soweit nichts anderes vermerkt ebenfalls stets auch gemeint ist eine solche erfindungsgemäße Probenahmevorrichtung, deren Probenkammer als ein die Probenahmevorrichtung durchmessender Kanal ausgebildet ist. Die Probenahmevorrichtung besitzt somit einen Probeneinlass der Probenkammer und, an einem weiteren Ende des die Probenkammer bildenden Kanals, einen Probenauslass. Der Probenauslass ermöglicht ein Austretenlassen einer Probe durch die Probenahmevorrichtung hindurch in einen Raum außerhalb der Probenahmevorrichtung. Dadurch wird es beispielsweise ermöglicht, die Probenahmevorrichtung in einer Schlaufe anzuordnen, auf deren einer Seite eine Probe aus einem Mediumhauptkörper eingeführt und auf deren anderer Seite eine die Probenahmevorrichtung durchlaufen habende Probe zurück in den Mediumhauptkörper ausgestoßen wird. Weitere vorteilhafte Ausgestaltungen von Probenahmevorrichtungen und Probenahmesystemen, die mit einer erfindungsgemäßen Probenahmevorrichtung mit kanalförmig ausgestalteter Probenkammer möglich sind, werden weiter unten insbesondere als erfindungsgemäße Probenahmesysteme, Probenahmeverfahren und Analyseverfahren beschrieben.

Der Fachmann versteht, dass bei einer kanalförmigen Ausführung der Probenkammer das Trennmedium nicht die gesamte Länge des Kanals abdecken muss. Ausreichend ist es bereits, wenn dasTrennmedium in der Probenkammer lediglich in einem ausreichend großen Abschnitt vorliegt, so dass die Probenkammer und die Analytaufnahmekammer durch das Trennmedium voneinander getrennt sind.

Die erfindungsgemäße Probenahmevorrichtung ist vorzugsweise ausgestaltet zum Anbringen an einem medizinischen Probenahmezugang, vorzugsweise einer Hohlnadel und insbesondere einer Kanüle, einem Katheter, einem Port, einem Sauger, einer Drainage, einem Reservoir und/oder einem Konnektor bzw. Verbinder. Die Probenahmevorrichtung ist dabei zweckmäßigerweise ausgestaltet zum form- und/oder kraft- und/oder reibschlüssigen Verbinden mit dem Probenahmezugang, also vorzugsweise dem medizinischen Probenahmezugang. Besonders bevorzugt ist die Probenahmevorrichtung form-, kraft- und/oder reibschlüssig verbindbar mit einem Luer-Anschluss, einem Bajonett-Anschluss und/oder einem Schraubverschluss. Derartige Anschlüsse sind in der Medizintechnik gebräuchlich und standardisiert. Die erfindungsgemäße Probenahmevorrichtung kann somit vorteilhafterweise an eine Vielzahl von Probenahmezugängen unterschiedlicher Hersteller angebracht und betrieben werden.

Vorzugsweise ist die Probenahmevorrichtung ausgestaltet zum Anordnen der Analytaufnahmekammer
- im Inneren eines zu beprobenden Mediumhauptkörpers durch einen Probenahmezugang hindurch,
- in einem Probenahmezugang,
- an einem bezogen auf einen zu beprobenden Mediumhauptkörper abgewandten Ende eines Probenahmezugangs, und/oder
- in einer mit einem Probenahmezugang verbundenen Leitung außerhalb eines zu beprobenden Mediumhauptkörpers.

Wenn mit der Probenahmevorrichtung also beispielsweise ein Patient beprobt werden soll, so dass beispielsweise sein Blutkreislauf den Mediumhauptkörper bildet, so kann die Probenahmevorrichtung ausgestaltet sein zum Anordnen der Analytaufnahmekammer
- im Inneren des Patienten, also "vor" dem Probenahmezugang in einem Blutgefäß,
- in einer Kanüle, anderen Hohlnadel oder einem Katheter,
- außerhalb des Patienten, also "hinter" dem Probenahmezugang, beispielsweise an einer Luer-Verbindung eines Probenahmezugangs, und/oder
- in einer an einen Probenahmezugang angeschlossenen Leitung, beispielsweise an einem Konnektor oder einem Reservoir.

Entsprechendes gilt, wenn nicht der Blutkreislauf, sondern ein anderer Teil eines Menschen oder Tieres oder eines anderen Mediums beprobt werden soll.

Statt mit dem Probenahmezugang form-, kraft- und/oder reibschlüssig verbindbar zu sein, insbesondere statt zum Anbringen an einem medizinischen Probenahmezugang ausgestaltet zu sein, kann die erfindungsgemäße Probenahmevorrichtung mit dem Probenahmezugang auch einstückig verbunden sein. Die Probenahmevorrichtung kann dann insbesondere so in einem medizinischen Probenahmezugang, insbesondere einer Kanüle oder anderen Hohlnadel, einem Katheter, einem Port, einem Sauger, einer Drainage, einem Reservoir und/oder einem Konnektor angeordnet sein, dass sie bei bestimmungsgemäßem Gebrauch des Probenahmezugangs im Inneren des Patienten, also beispielsweise an der Spitze des Probenahmezugangs, im Probenahmezugang oder "hinter" dem Probenahmezugang, also außerhalb des Patienten, angeordnet ist. Besonders bevorzugt ist es dabei, wenn die Probenahmevorrichtung einstückig Bestandteil einer Hohlnadel ist, insbesondere einer Kanüle und/oder eines Katheters. Durch Setzen einer solchen erfindungsgemäßen Probenahmevorrichtung kann in einem Schritt sowohl ein Probenahmezugang geschaffen als auch die Probenahmevorrichtung selbst im Patienten angeordnet werden, ohne dass dabei zu besorgen wäre, dass ein fester Körper des Patienten wie beispielsweise eine Gefäßwand am Trennmedium ganz oder teilweise anliegt und so den Übertritt eines Analyten aus einer Probe in die Analytaufnahmekammer behindert.

Die erfindungsgemäße Probenahmevorrichtung, sei sie einstückig verbunden oder, was bevorzugt ist, anbringbar an einem Probenahmezugang, ist vorzugsweise ausgestaltet zum Aufnehmen einer Probe von Körperflüssigkeit, wobei die Körperflüssigkeit vorzugsweise ausgewählt ist aus Blut, Blutplasma, Lymphe, Gewebeflüssigkeit, Liquor cerebrospinalis, Synovia, Magensaft, Galle und Urin. Die erfindungsgemäße Probenahmevorrichtung ist auf solche Proben jedoch nicht beschränkt, sondern ist in weiteren bevorzugten Ausführungsformen ausgestaltet zum Aufnehmen einer Probe einer Zellkultur, vorzugsweise einer Flüssig-Zellkultur, und/oder eines flüssigen und/oder gasförmigen biologischen oder chemischen Reaktorinhalts. Je nach zu untersuchender Probe und gegebenenfalls auch in Abhängigkeit von dem jeweils gewählten Probenahmeort wird der Fachmann beispielsweise die Größe der Probenahmekammer sinnfällig wählen.

Im Sinne der vorliegenden Erfindung ist ein Sensor jede Vorrichtung oder Gruppe von Vorrichtungen, die in Abhängigkeit von der Anwesenheit oder Menge eines Analyten ein Messsignal erzeugt. Ein Sensor kann insbesondere ausgewählt sein aus der Gruppe bestehend aus elektrochemischen Sensoren, optischen Sensoren, amperometrischen Sensoren, Leitfähigkeitssensoren, potentiometrischen Sensoren, Biosensoren, Sauerstoffsensoren, enzymatischen Sensoren, Spektralfotometer, NIR-Analysatoren, IR-Analysatoren, Fluoreszenzfotometer und Probenaufgeber für Gaschromatographen oder HPLC-Geräte.

Der Fachmann wird das Trennmedium zweckmäßig wählen. Erfindungsgemäß bevorzugt sind solche Probenahmevorrichtungen, bei denen das Trennmedium eine Membran ist, vorzugsweise eine Dialysemembran. Soweit im Rahmen dieser Beschreibung von einem Trennmedium die Rede ist, so ist damit immer auch, also zusätzlich, eine Ausführungsform gemeint, deren Trennmedium eine Membran ist, vorzugsweise eine Dialysemembran. Dialysemembranen im Sinne der Erfindung sind Membranen, die den Durchtritt chemischer Verbindungen lediglich bis zu einer gewissen Größe der Verbindung gestatten. Beispielsweise ermöglichen es Dialysemembranen, Ionen wie beispielsweise Na⁺ zu trennen von anderen geladenen oder ungeladenen Substanzen größerer Größe, beispielsweise Peptide oder Proteine. In Ansehung des jeweiligen Analyten, und gegebenenfalls auch des jeweiligen Sensors kann der Fachmann eine geeignete Membran, insbesondere eine geeignete Dialysemembran, auswählen.

Wenn das Trennmedium eine Membran (nachfolgend auch als "Probenmembran" bezeichnet) ist, dann ist das Material der Probenmembran vorzugsweise ausgewählt aus der Gruppe bestehend aus Zellulose und deren Derivaten, insbesondere Zelluloseacetat, PTFE, Polykarbonat, Polypropylen, Polyamiden, Polyestern, Polyethersulfonen und Polysulfonen.

Ferner bevorzugt ist eine erfindungsgemäße Probenahmevorrichtung, deren Trennmedium und insbesondere deren Probenmembran ausgestaltet ist zum Durchtretenlassen eines Analyten ausgewählt aus Glukose, Laktose, Laktat, Na⁺, K⁺, Cl⁻, H₃O⁺, O₂, CO₂, Ammonium, Ammoniak, Methanol, Ethanol, Formiat, Acetat, Glutamin, Glutamat, Harnstoff, Harnsäure, Phosphat, Antikörpern, Wachstumsfaktoren, Hormonen, Medikamenten und insbesondere Narkotika.

Der Zuleitungskanal und/oder der Ableitungskanal einer erfindungsgemäßen Probenahmevorrichtung weist an der Analytaufnahmekammer oder an seiner engsten Stelle in der Probenahmevorrichtung einen Innendurchmesser von 0,01 bis 2 mm auf, vorzugsweise von 0,1 bis 1,5 mm und besonders bevorzugt von 0,25 bis 0,5 mm. Mit solchen Innendurchmessern lassen sich auch in der klinischen Anwendung bei Verwendung wässriger Transportmedien so hohe Drücke aufbauen, dass ein rascher und im Wesentlichen pfropfenförmiger Transport des Transportmediums aus der Analytaufnahmekammer in Richtung auf und in einen Sensor erzielt werden kann, ohne dass zu besorgen wäre, dass die zur Leitung des Transportmediums verwendeten Leitungen von der Probenahmevorrichtung abreißen oder das Trennmedium, insbesondere die Probenmembran und insbesondere eine Dialysemembran, reißen könnte.

Alternativ oder zusätzlich zu diesen Innendurchmesser-Abmessungen ist es bevorzugt, dass die Fläche desjenigen Trennmediumabschnittes, der die Probenkammer mit der Analytaufnahmekammer verbindet (nachfolgend "Membranfenster" genannt), 0,5 bis 350 mm² beträgt, vorzugsweise 1 bis 50 mm² und besonders bevorzugt 2 bis 35 mm². Diese Membranfenstergrößen sind insbesondere vorteilhaft angepasst an die in Kathetern und Kanülen zur Verfügung stehenden Abmessungen und erlauben doch einen raschen Analytdurchtritt aufgrund der überraschend groß dimensionierbaren Fläche des Membranfensters. Bei ersten Erprobungen besonders bewährt hat sich eine Membranfensterfläche von 34 mm². Dabei betrug der Durchmesser des im Querschnitt kreisförmigen Ableitungskanals 0,23-0,26 mm, vorzugsweise 0,25 mm.

Das Verhältnis der Fläche des Membranfensters zur Mindest-Querschnittsfläche des Ableitungskanals beträgt in einer bevorzugten Ausführungsform höchstens 400, vorzugsweise höchstens 200 und besonders bevorzugt 40 bis 80. In einer anderen Ausführungsform bevorzugt beträgt das Verhältnis mehr als 400, vorzugsweise 450 bis 1000 und besonders bevorzugt 500 bis 750.

Die erfindungsgemäße Probenahmevorrichtung umfasst vorzugsweise ferner eine bewegliche Verschlussmembran zum Verringern und/oder Verschließen eines Volumens der Probenkammer oder des Probenahmezugangs. Dabei umfasst die Probenahmevorrichtung zweckmäßigerweise ferner einen Verschluss-Steuerungskanal zum Ausüben eines Druckes zum Bewegen der Verschlussmembran. Durch die Verschlussmembran wird auf vorteilhaft einfache Weise möglich, die Probenkammer vom zu beprobenden Mediumhauptkörper abzutrennen, indem ein in Mediumsfließrichtung vor der Analytaufnahmekammer (also vor dem Membranfenster) gelegenes Volumen ausgefüllt wird. Auf diese Weise kann eine ruhende Probe in der Probenkammer gewährleistet werden oder das Totvolumen des Probenahmezugangs verrringert werden. Die Verschlussmembran kann aber auch zusätzlich oder alternativ so ausgestaltet werden, dass sie in Mediumsfließrichtung hinter der Analytaufnahmekammer (also hinter dem Membranfenster) ein Volumen der Probenkammer freigeben oder verringern kann. Damit wird es möglich, eine Probe in die Probenkammer hineinzusaugen oder aus der Probenkammer auszustoßen.

Beispielsweise kann der Probenahmezugang bei Verwendung eines Multilumenkatheters so ausgestaltet sein, dass das mit der Probennahmevorrichtung verbundene Lumen mittels eines im Innern des Lumens eingebrachten Membranschlauches reversibel durch Aufblasen verschlossen werden kann. Dadurch kann zwischen den Mediumbeprobungen das Totvolumen verringert und ein sicherer Verschluss der Probenahmezuführung gewährleistet werden. Entsprechende Vorrichtungen sind auch für Kanülen oder andere Probenahmezugänge darstellbar.

Ferner bevorzugt ist eine solche erfindungsgemäße Probenahmevorrichtung, die einen Alternativauslass umfasst zum Austretenlassen von in der Probenkammer aufgenommenem Medium. Eine solche Probenahmevorrichtung besitzt eine kanalförmig ausgestaltete Probenkammer mit drei Ausgängen, nämlich dem Probeneinlass, dem oben beschriebenen weiteren Ausgang des Kanals und dem zuvor beschriebenen Alternativauslass. Insbesondere in Verbindung mit einer Verschlussmembran ist es dann möglich, eine Probe aus dem Kanalausgang und/oder dem Alternativauslass aus der Probenahmevorrichtung austreten zu lassen. Damit ist eine besonders einfache Möglichkeit zum Kalibrieren mit Hilfe einer erfindungsgemäßen Probenahmevorrichtung möglich: Dazu wird in einem ersten Schritt eventuell in der Probenkammer vorliegendes Probenmaterial ausgetrieben durch Beaufschlagen der Probenkammer mit einem Kalibriermedium. Wenn die Probenahmevorrichtung über eine Verschlussmembran verfügt, so wird diese zweckmäßigerweise verschlossen, um den Eintritt von zu beprobendem Medium in die Probenkammer zu verhindern. Eine eventuell in der Probenkammer vorliegende Probe wird dann durch das Kalibriermedium über den Alternativauslass aus der erfindungsgemäßen Probenahmevorrichtung ausgetrieben. Verfügt die Probenahmevorrichtung nicht über eine Verschlussmembran, so ist es zweckmäßig, am Alternativauslass einen verglichen mit dem Druck des Kalibriermediums und dem Druck des zu beprobenden Mediums geringeren Druck anzulegen, so dass durch den Alternativauslass eine Mischung aus Kalibriermedium und dem zu beprobenden Medium austritt. Wenn der Alternativauslass zwischen dem Probeneinlass und der Analytaufnahmekammer (also dem Membranfenster) angeordnet ist, dann wird so sichergestellt, dass einerseits kein Kalibriermedium in das zu beprobende Medium (dem Mediumhauptkörper) hineingepresst wird - denn es wird hier noch immer zu beprobendes Medium durch den Alternativauslass abgesaugt -, andererseits aber auch kein zu beprobendes Medium zum Membranfenster gelangt - aus der Richtung strömt ja das Kalibriermedium ein.

Erfindungsgemäß wird ferner ein Probenahmesystem angegeben, umfassend
- eine Probenahmevorrichtung,
- einen Sensoranschluss zum Anschließen eines Sensors zum Nachweisen eines Analyten in einem Transportmedium,
- eine Zuleitung, fluidleitend verbunden mit dem Zuleitungskanal der Probenahmevorrichtung,
- eine Ableitung, fluidleitend verbunden mit dem Ableitungskanal der Probenahmevorrichtung und dem Sensoranschluss,
- eine erste Pumpe zum Transportieren eines Mediums durch die Ableitung zum Sensoranschluss.

Die Probenahmevorrichtung ist, soweit nichts anderes gesagt ist, eine oben beschriebene erfindungsgemäße Probenahmevorrichtung. Alternativ dazu kann im erfindungsgemäßen Probenahmesystem auch eine Probenahmevorrichtung eingesetzt werden, deren Analytaufnahmekammer nicht eine innenliegende Probenkammer umgibt, sondern die durch ein auf dem Außenrand der Probenahmevorrichtung angeordnetes Membranfester mit dem zu beprobenden Medium verbunden ist. Eine solche Probenahmevorrichtung ist vorzugsweise in einer Hohlnadel angeordnet, vorzugsweise einer Kanüle oder einem Katheter.

Ein erfindungsgemäßes Probenahmesystem verwirklicht die mit der erfindungsgemäßen Probenahmevorrichtung erreichbaren Vorteile. Insbesondere erlaubt das erfindungsgemäße Probenahmesystem gegebenenfalls analytbeladenes Transportmedium aus der Analytaufnahmekammer der Probenahmevorrichtung zu einem Sensoranschluss und, wenn ein Sensor angeschlossen ist, auch zum Untersuchen des Transportmediums in den Sensor zu transportieren.

Die erste Pumpe des erfindungsgemäßen Probenahmesystems kann insbesondere angeordnet sein an oder in Medientransportrichtung vor der Zuleitung. In diesem Fall erzeugt die Pumpe einen Druck, um Transportmedium durch die Zuleitung, die Analytaufnahmekammer und die Ableitung zum Sensoranschluss zu drücken. Die Pumpe kann jedoch auch angeordnet sein zwischen dem Ableitungskanal der Probenahmevorrichtung und dem Sensoranschluss, vorzugsweise an der Ableitung. Bei einer solchen Anordnung zwischen dem Ableitungskanal der Probenahmevorrichtung und dem Sensoranschluss erzeugt die erste Pumpe auf der einen Seite einen Unterdruck, um Transportmedium durch die Zuleitung in die Analytaufnahmekammer der Probenahmevorrichtung ein- und aus ihr herauszusaugen, während sie auf der anderen Seite einen Überdruck erzeugt, um Transportmedium durch die Ableitung zum Sensoranschluss und gegebenenfalls in einen Sensor zu transportieren. In einer dritten Ausführungsform kann die erste Pumpe in Transportrichtung hinter dem Sensoranschluss und insbesondere hinter einem Sensor angeschlossen sein. In diesem Falle erzeugt die erste Pumpe in ihrem Betrieb einen Unterdruck, um Transportmedium durch die Zuleitung, die Analytaufnahmekammer und die Ableitung zum Sensoranschluss und vorzugsweise durch den Sensor zu saugen.

Die Anordnung der ersten Pumpe an oder vor der Zuleitung ist besonders bevorzugt in solchen Fällen, in denen es auf den raschen Transport des Transportmediums ankommt. Durch Anlegen eines geringen Überdrucks während des Transports wird erreicht, dass sämtliche zum Transport benötigten Leitungen und auch das Trennmedium, insbesondere die Trennmembran der Probenkammer, aus Sicht des Transportmediums ausgedehnt werden, so dass die Leitungen nicht wie bei Vorliegen eines Unterdrucks möglich kollabieren.

In solchen Fällen, in denen eine Kontamination des zu beprobenden Mediums mit höchster Priorität vermieden werden soll, ist es bevorzugt, die erste Pumpe zwischen dem Ableitungskanal und dem Sensoranschluss oder in Mediumstransportrichtung hinter dem Sensoranschluss und gegebenenfalls dem Sensor anzuordnen. Auf diese Weise kann auch bei einem Beschädigen des Trennmediums und insbesondere einer Trennmembran der Eintritt von Transportmedium in das zu beprobende Medium vorteilhaft einfach verhindert werden, indem in der Analytaufnahmekammer beim Transport des Transportmediums im Verhältnis zum zu beprobenden Medium ein Unterdruck angelegt wird. In diesem Fall wird lediglich Transportmedium durch und aus der Analytaufnahmekammer herausgesaugt. Wenn das Trennmedium beschädigt ist, so würde Transportmedium und zu beprobendes Medium aus der Analytaufnahmekammer abgesaugt.

Ein erfindungsgemäß bevorzugtes Probenahmesystem umfasst ferner eine Probenahmeleitung und mit dieser verbunden eine zweite Pumpe zum Pumpen einer Probe in die Probenkammer. In der weiteren Beschreibung dieser Erfindung ist ein solches Probenahmesystem jeweils auch gemeint, soweit nichts anderes angegeben ist. Die weiteren Beschreibungen des erfindungsgemäßen Probenahmesystems betreffen also sowohl ein Probenahmesystem ohne als auch ein Probenahmesystem mit zweiter Pumpe. Wenn die Probenahmevorrichtung des erfindungsgemäßen Probenahmesystems keine erfindungsgemäße Probenahmevorrichtung ist, sondern im Inneren einer Hohlnadel angeordnet ist, so ist die Probenahmeleitung verbunden mit der Hohlnadel zum Pumpen einer Probe in das dann als Probenkammer wirkende Innenlumen der Hohlnadel.

Aus der WO 2008/059050 A2 ist eine Venenverweilkanüle mit einer Analytaufgabekammer bekannt, wobei die Analytaufgabekammer mit einer inneren Ausnehmung der Kanüle verbunden ist, um ein in der Ausnehmung befindliches Medium zu beproben. Das Dokument lehrt jedoch nicht, die Probenkammer mit einem Trennmedium zum Durchtretenlassen des Analyten in die Analytaufgabekammer zu umgeben, und es lehrt auch nicht, ein Medium in die innere Ausnehmung einzusaugen. Es lehrt ferner nicht, in Abhängigkeit von dieser Einsaugung eine Pumpvorrichtung zu steuern (dazu sogleich mehr), um eine Probe eines gegebenenfalls analythaltigen Transportmediums zu erhalten. Das Dokument gibt zudem keinen Hinweis darauf, dass diese Venenverweilkanüle weiterentwickelt werden kann, um die obigen Aufgaben der Erfindung zu lösen. Vielmehr wird lediglich der einzelne Venenverweilkatheter als solcher beiläufig erwähnt.

Die zweite Pumpe erlaubt es vorteilhafterweise, eine Probe eines zu beprobenden Mediums in die Probenkammer einzusaugen. Der Fachmann versteht, dass dieses Einsaugen das Ziel hat, einen Analyten aus der Probe durch das Trennmedium, insbesondere eine Trennmembran, in die Analytaufnahmekammer der Probenahmevorrichtung eintreten zu lassen, um ein darin befindliches Transportmedium mit dem Analyten zu beladen. Der Fachmann kann die Menge des einzuleitenden Mediums und damit die Größe der Probe in Abhängigkeit vom Volumen der Probenkammer und der Anordnung der Analytaufnahmekammer leicht zum Bewältigen seines jeweiligen Messproblems entsprechend auswählen. Besonders bevorzugt ist es, die Probenkammer der r Probenahmevorrichtung vollständig mit dem zu beprobenden Medium auszufüllen.

Ebenfalls möglich und bevorzugt ist es, mit Hilfe der zweiten Pumpe mehr Medium durch die Probenahmeleitung einzusaugen als die Probenahmevorrichtung in ihrer Probenkammer fassen kann. Es tritt dann etwas zu beprobendes Medium in die Probenahmeleitung aus. Auf diese Weise ist es möglich, die Probenkammer mit dem zu beprobenden Medium vollständig auszuspülen. Insbesondere vermeidet ein solches Vorgehen, dass nur ein solches Medium in die Probenkammer eingesaugt wird, das zwischen dem Mediumhauptkörper und dem Membranfenster als Totvolumen abgestanden ist. Wenn die Probenahmevorrichtung beispielsweise in einer Hohlnadel, insbesondere einem Katheter oder einer Kanüle angeordnet ist, so könnte es bei einem zu geringen Probenahmevolumen dazu kommen, dass lediglich solches Medium in die Probenkammer gerät, das in der Spitze der Hohlnadel in Saugrichtung vor der Probenkammer und damit nicht im eigentlichen Mediumstrom angeordnet war. Ein solches Mediumvolumen kann eine andere Zusammensetzung als das Medium des Mediumhauptkörpers besitzen, da in einem weitgehend ruhenden Mediumvolumen der Hohlnadelspitze Entmischungen und beispielsweise Gerinnungsvorgänge ablaufen können. Indem jedoch ein ausreichend groß gewähltes Mediumvolumen in die Probenkammer und Probenahmeleitung eingesogen wird, kann vermieden werden, nur solche Medien-Totvolumina zu beproben. Zum Vermeiden solcher Totvolumina ist es ebenfalls zweckmäßig, zusätzlich eine bewegliche Verschlussmembran wie oben beschrieben einzusetzen. Überraschenderweise kann aber auch reproduzierbar eine Mischung von Probe und Medien-Totvolumen gemessen werden.

Ein erfindungsgemäßes Probenahmesystem, also ein Probenahmesystem mit einer ersten und vorzugsweise auch einer zweiten Pumpe wie oben beschrieben, umfasst vorzugsweise ferner einen Transportmediumvorrat und/oder einen Kalibriermediumvorrat und/oder ein Spülmediumvorrat. Der jeweilige Mediumvorrat bzw. zwei oder alle Mediumvorrate sind mit der Zuleitung fluidleitend verbunden oder verbindbar. Umfasst das Probenahmesystem auch eine Probenahmeleitung, so ist es bevorzugt, wenn einer, mehrere oder alle der Mediumvorrate fluidleitend verbunden oder verbindbar ist bzw. sind mit der Probenahmeleitung.

Ein Transportmedium im Sinne dieser Erfindung ist ein vorzugsweise flüssiges und insbesondere bevorzugt wässriges, gegebenenfalls aber auch ein gasförmiges Medium, das zum Transportieren des zu bestimmenden Analyten an einen gewählten Sensor geeignet ist. Ein geeignetes Transportmedium kann der Fachmann in Ansehung des zu bestimmenden Analyten, des zu beprobenden Mediums, des Sensors und der Betriebsparameter des erfindungsgemäßen Probenahmesystems, beispielsweise der Temperatur oder den zugehörigen Leitungsdurchmessern, leicht auswählen. Wenn das zu beprobende Medium eine Körperflüssigkeit ist, insbesondere Blut, Blutplasma, Lymphe, Gewebeflüssigkeit oder Liquor cerebrospinalis, so ist es bevorzugt, dass das Transportmedium eine isotonische Kochsalzlösung ist, wobei eine Lösung von 0,9 Gew.-% NaCl in Wasser besonders bevorzugt ist. Ebenfalls bevorzugt sind Vollelektrolytlösungen mit physiologischer Osmolarität. Transportmedien, die an die physiologische Osmolarität angepasst sind, insbesondere eine der physiologischen Osmolarität nahezu oder vollständig gleiche Osmolarität besitzen, ermöglichen es, eine Körperflüssigkeit und insbesondere die genannten Körperflüssigkeiten zu beproben, ohne den Durchtritt des Analyten durch gleichzeitigen übermäßigen Durchtritt von Wasser durch das Trennmedium und insbesondere die Trennmembran zu verfälschen.

Das Transportmedium kann weitere Substanzen enthalten, insbesondere in Abhängigkeit vom zu bestimmenden Analyten und dem hierzu verwendeten Sensor. Beispielsweise kann das Transportmedium Reagenzien enthalten, die mit dem nachzuweisenden Analyten reagieren, wobei im Sensor das Reaktionsergebnis bestimmt wird. Beispiele solcher weiterer Substanzen sind Farbstoffe und insbesondere Fluoreszenzfarbstoffe.

Ein Kalibriermedium im Sinne dieser Erfindung ist ein Medium, das eine vorbekannte Konzentration eines oder mehrerer zu bestimmender Analyten enthält. Das Kalibriermedium dient dann hauptsächlich dazu, einen Sensor auf diese Konzentration(en) zu kalibrieren. Dazu kann das Kalibriermedium einem Sensor zugeleitet werden durch (a) Einführen des Kalibriermediums in die Probenkammer zum Beaufschlagen eines Transportmediums mit dem Analyten und anschließend Transport des beaufschlagten Transportmediums zum Sensorausgang und vorzugsweise zum Sensor, (b) Durchspülen der Analytaufnahmekammer mit Kalibriermedium und Transportieren des Kalibriermediums zum Sensorausgang und vorzugsweise zum Sensor, und/oder (c) unmittelbares Beaufschlagen des Sensors oder des Sensorausgangs mit dem Kalibriermedium, also unter Umgehen der Probenahmevorrichtung.

Ein Spülmedium im Sinne dieser Erfindung ist ein Medium, das zum Durchspülen der Probenkammer und/oder Analytaufnahmekammer eingerichtet ist, vorzugsweise um diese von Kalibriermedium und/oder Transportmedium, insbesondere analyhaltigem Transportmedium, zu reinigen. Das Spülmedium und auch das Kalibriermedium besitzt vorzugsweise die gleiche oder annäherungsweise gleiche Osmolarität wie das Transportmedium. Wird das Spülmedium und/oder das Kalibriermedium in die Probenkammer eingeführt, so dass es von dort aus in einen Patienten (Mensch oder Tier) gelangen kann, so ist es bevorzugt, ein physiologisch kompatibles Medium als Kalibriermedium und/oder Spülmedium zu verwenden, vorzugsweise also isotonische Kochsalzlösung oder ein Transportmedium wie oben beschrieben.

Erfindungsgemäß besonders bevorzugt ist ein solches Probenahmesystem, bei dem die erste Pumpe angeordnet ist zwischen (a) dem Transportmediumvorrat und/oder dem Kalibriermediumvorrat und/oder dem Spülmediumvorrat und (b) dem Zuleitungskanal der Probenahmevorrichtung zum Transportieren von Medium vom jeweiligen Mediumvorrat durch die Zuleitung in den Zuleitungskanal. Die mit einer solchen Anordnung der ersten Pumpe verbundenen Vorteile sind bereits oben beschrieben.

Ein erfindungsgemäßes Probenahmesystem umfasst vorzugsweise ferner eine Bypassleitung zum fluidleitenden Verbinden des Transportmediumvorrats und/oder des Kalibriermediumvorrats und/oder des Spülmediumvorrats mit dem Sensoranschluss ohne Passieren der Analytaufnahmekammer der Sensorvorrichtung. In einer solchen Ausgestaltung ist es vorteilhaft möglich, analytfreies Transportmedium, Spülmedium und/oder Kalibriermedium unmittelbar zum Sensorausgang und, wenn ein Sensor vorhanden ist, zum Sensor selbst zu leiten, beispielsweise um den Sensor zu spülen oder zu kalibrieren. Falls das Kalibriermedium und/oder das Spülmedium physiologisch unverträglich sein sollte, bietet diese Ausgestaltung ferner den Vorteil, das Kalibriermedium und/oder das Spülmedium soweit wie möglich von der Probenahmevorrichtung fernzuhalten , so dass auch bei einer versehentlichen Beschädigung des Trennmediums, insbesondere der Trennmembran, eine Kontamination der Probe bzw. des Mediumhauptkörpers mit dem Kalibriermedium und/oder dem Spülmedium ausgeschlossen werden kann.

Ferner bevorzugt ist es, wenn die Bypassleitung durch einen schaltbaren Anschluss mit der Ableitung verbunden ist. Ein solcher schaltbarer Anschluss bietet eine zusätzliche Sicherheit gegen ein versehentliches Vermischen von durch die Ableitung transportiertem gegebenenfalls analythaltigem Transportmedium mit noch in der Bypassleitung vorhandenem Transportmedium, Kalibriermedium und/oder Spülmedium.

Bevorzugt ist zudem ein erfindungsgemäßes Probenahmesystem umfassend einen schaltbaren Anschluss zum alternativen fluidleitenden Verbinden des Transportmediumvorrats und/oder des Kalibriermediumvorrats und/oder des Spülmediumvorrats mit entweder der Zuleitung oder der Bypassleitung. Ein solcher schaltbarer Anschluss kann zusätzlich oder alternativ zu einem schaltbaren Anschluss zum Verbinden der Bypassleitung mit der Ableitung vorgesehen sein. Durch den schaltbaren Anschluss zum Verbinden des jeweiligen Mediumvorrats mit der Zuleitung oder Bypassleitung wird es auf vorteilhaft einfache Weise möglich, Kontaminationen des Transportmediums, des Kalibriermediums und/oder des Spülmediums mit eventuell in der Bypassleitung bzw. der Zuleitung vorliegendem Kalibriermedium, Spülmedium und/oder Transportmedium zu verhindern. Beispielsweise kann so sichergestellt werden, dass Kalibriermedium stets nur in die Bypassleitung einströmt, während Transport- und Spülmedium ohne Kontakt zum Kalibriermedium durch die Zuleitung geleitet werden können.

Ein erfindungsgemäßes Probenahmesystem ist auch dann bevorzugt, wenn es eine Ausflussleitung zum fluidleitenden Verbinden der Ableitung über den Sensoranschluss und gegebenenfalls ein Sensor mit der Probenahmeleitung umfasst. Auf diese Weise wird es möglich, ein durch den Sensor vermessenes Medium statt in einen Medienabfall durch die Probenahmeleitung in das zu beprobende Medium einzuführen und so gegebenenfalls zu entsorgen. Wenn das zu beprobende Medium ein Medium eines menschlichen oder tierischen Patienten ist, insbesondere eine Körperflüssigkeit, dann ist es zweckmäßig, das erfindungsgemäße Probenahmesystem steril auszugestalten. Dies gilt insbesondere dann, wenn wie soeben beschrieben eine Ausflussleitung vorgesehen ist zum fluidleitenden Verbinden der Ableitung über den Sensoranschluss und gegebenenfalls ein Sensor mit der Probenahmeleitung.

Dabei ist ein Probenahmesystem besonders bevorzugt, das einen schaltbaren Anschluss umfasst zum alternativen fluidleitenden Verbinden (a) der Ableitung mit der Ausflussleitung gegebenenfalls über einen Sensor, oder (b) der Ableitung mit einer Abfallleitung gegebenenfalls über einen Sensor zum Leiten von aus der Ableitung gegebenenfalls durch den Sensor austretendem Medium zu einer Mediumentsorgung. Auf diese Weise kann ausgewählt werden, ob ein durch den Sensoranschluss und gegebenenfalls den Sensor austretendes Medium entsorgt werden oder dem zu beprobenden Medium zugeführt werden soll.

Wenn ein erfindungsgemäßes Probenahmesystem -wie es bevorzugt ist- eine Probenahmeleitung und eine zweite Pumpe umfasst, so ist es besonders bevorzugt, wenn das Probenahmesystem zusätzlich einen schaltbaren Anschluss zum Verbinden des Transportmediumvorrats und/oder des Kalibriermediumvorrats und/oder des Spülmediumvorrats mit der Probenahmeleitung über die zweite Pumpe umfasst. Dadurch ist es möglich, die Probenkammer der Probenahmevorrichtung mit einem Transportmedium, einem Kalibriermedium und/oder einem Spülmedium zu beaufschlagen. Insbesondere bevorzugt ist es, wenn die Probenkammer mit Kalibriermedium und/oder Spülmedium wie beschrieben beaufschlagt werden kann. Bei Beaufschlagen der Probenkammer mit Kalibriermedium ist es wie eingangs beschrieben möglich, ein Transportmedium in der Analytaufnahmekammer mit einem Analyten aus dem Kalibriermedium zu beaufschlagen, so dass entsprechend beaufschlagtes Transportmedium durch die Ableitung zum Sensoranschluss und zweckmäßigerweise zum Sensor zu transportieren. Das am Sensor bestimmte Signal des Analyten kann dann korreliert werden mit einer bekannten Analytkonzentration des Kalibriermediums. Auf diese Weise kann ein sehr präziser Rückschluss auf die tatsächliche Analytkonzentration des zu beprobenden Mediums erreicht werden, da mögliche Messfehlerquellen, wie sie sich durch den notwendigerweise zu bewirkenden Übertritt des Analyten aus dem Kalibriermedium durch das Trennmedium, insbesondere eine Trennmembran, in die Analytaufnahmekammer ergeben, ausgeschlossen oder deutlich verringert werden können. Besonders bevorzugt umfasst dann ein erfindungsgemäßes Probenahmesystem eine Probenahmevorrichtung mit einem Alternativauslass, wobei der Alternativauslass besonders bevorzugt in Fließrichtung des Kalibriermediums und/oder Transportmediums und/oder Spülmediums hinter dem Membranfenster angeordnet ist. Dies ermöglicht es wie eingangs beschrieben, die Probenkammer mit Kalibriermedium und/oder Spülmedium und/oder Transportmedium zu spülen und das zum Spülen verwendete Medium statt in den Vorrat des zu beprobenden Mediums durch den Alternativauslass austreten zu lassen. Dabei ist es wie ebenfalls oben beschrieben bevorzugt, eine Probenahmevorrichtung einzusetzen mit einer Verschlussmembran, oder das Austreten eines Mediums aus dem Alternativauslass durch eine weitere Pumpe zu steuern.

Das erfindungsgemäße Probenahmesystem besitzt vorzugsweise eine Steuerung, verbunden mit der zweiten Pumpe, um die zweite Pumpe so zu steuern, dass
- Schritt 1:: eine Probe in die Probenkammer eingesaugt wird,
- Schritt 2:: die eingesaugte Probe eine vorgewählte Zeit lang in der Probenkammer vorliegt, und anschließend
- Schritt 3:: die eingesaugte Probe aus der Probenkammer ausgestoßen wird.

Ein so gesteuertes Probenahmesystem erlaubt es auf überraschend einfache und vorteilhafte Weise, reproduzierbare Analytkonzentrationen in der gegebenenfalls analythaltigen Probe zu erreichen, wobei diese Konzentration in kalibrierbarem Zusammenhang mit der Analytkonzentration in dem zu untersuchenden Medium steht. Besonders vorteilhaft wirkt sich aus, dass bei Anwendung des Probenahmesystems an einem Lebewesen, insbesondere an einem Menschen, die Probenahmevorrichtung über einen längeren Zeitraum in dem Lebewesen eingeführt verbleiben kann, wobei der Einfluss unwillkürlicher Bewegungen oder Blutströmungsschwankungen gegenüber einer herkömmlich implantierten Mikrodialysesonde verringert wird. Ein weiterer Vorteil ist, dass ein wie oben beschriebenes Probenahmesystem die Probenahmeleitung als Schlaufe ausgebildet haben kann, so dass aus einem Mediumhauptkörper entnommene Probe nach Durchlaufen der Probenahmevorrichtung und gegebenenfalls Übertritt des Analyten in das Transportmedium dem Mediumhauptkörper erneut zugeführt werden kann. Ist die Probenahmevorrichtung beispielsweise am Blutstrom eines Patienten angeschlossen, so erlaubt es diese Ausgestaltung, die Entnahme von Blut ohne wesentlichen Blutverlust für den Patienten durchzuführen.

Wenn das erfindungsgemäße Probenahmesystem eine Hohlnadel umfasst wie oben beschrieben, indem also eine erfindungsgemäße Probenahmevorrichtung in oder an einer Hohlnadel angeschlossen ist oder eine nicht erfindungsgemäße Probenahmevorrichtung in eine Hohlnadel eingeführt ist, dann ist die Hohlnadel vorzugsweise eine Kanüle, eine Verweilkanüle, ein Katheter oder ein Verweilkatheter. Derartige Vorrichtungen sind insbesondere aus der Medizin bekannt; sie werden in großen Stückzahlen mit reproduzierbarer Beschaffenheit hergestellt und gelten als sicher für den Einsatz an einem Menschen. Besonders bevorzugt hat die Hohlnadel einen Innendurchmesser von 0,8 bis 1,3 mm. Durch geeignete Anordnung der Analytaufnahmekammer in der Hohlnadel kann so erreicht werden, dass zum sicheren Bedecken des Trennmediums und insbesondere einer Trennmembran am Membranfenster eine Probe des zu analysierenden Mediums von 50 bis 150 µl ausreichend ist, wobei bei einem Innendurchmesser der Hohlnadel von 0,96 mm ein Probenvolumen von 75 µl bei einem Abstand des nächsten Randes des Membranfensters zur Spitze der Hohlnadel von 3 mm bevorzugt ist. Entsprechend bevorzugt sind proportional erhöhte bzw. verringerte Probenvolumina bei einem erhöhten bzw. verringerten Innendurchmesser der Hohlnadel und/oder des Abstandes des Membranfensterrandes von der Hohlnadelspitze. Insbesondere bei Anwendung der erfindungsgemäßen Probenahmevorrichtung an einem Patienten kann so die Belastung des Patienten durch die Probenahme gering gehalten werden. Wenn die Hohlnadel einen anderen als einen kreisförmigen Querschnitt hat, beispielsweise einen ovalen oder rechteckigen Querschnitt, dann ist es bevorzugt, die obigen Angaben auf die jeweilige Hohlnadel so anzupassen, dass die Querschnittsfläche jeweils der Fläche eines runden Querschnitts mit den oben genannten Durchmessern entspricht. Die Ableitung und die Zuleitung und auch der Ableitungskanal und der Zuleitungskanal haben vorzugsweise einen Innendurchmesser von 0,15 bis 0,8 mm, wobei besonders bevorzugt die in der WO 2008/059050 genannten Durchmesser sind.

Wenn das Probenahmesystem eine Hohlnadel umfasst wie oben beschrieben, dann ist es bevorzugt, die Probenahmevorrichtung einstückig verbunden mit der Hohlnadel bereitzustellen. Die Hohlnadel enthält dann an einem vorgewählten Ort ein Membranfenster sowie eine die Probenkammer vorzugsweise umgebende Analytaufnahmekammer. Eine solche Ausgestaltung vermeidet auf vorteilhaft einfache Weise das Problem, ins Innere einer Hohlnadel und insbesondere gegen einen Blutfluss eine Probenahmevorrichtung separat einzuführen.

Alternativ dazu kann die Probenahmevorrichtung mit der Hohlnadel auch wie oben beschrieben lösbar verbunden sein. Diese Ausgestaltung ist insbesondere dann vorteilhaft und bevorzugt, wenn der Probenahmezugang zu dem zu beprobenden Medium für eine längere Zeit bestehen bleiben soll als die Probenahmevorrichtung im Kontakt mit dem zu beprobenden Medium bleiben soll. Beispielsweise kann es vorteilhaft sein, eine Kanüle für die Dauer von 4 Wochen bei einem Patienten zu setzen, die Probenahmevorrichtung jedoch wöchentlich auszutauschen.

Ein erfindungsgemäßes Probenahmesystem umfasst vorzugsweise eine solche Steuerung, die mit der ersten Pumpe verbunden ist, um diese so zu steuern, dass in Schritt 2:
Schritt a): Transportmedium in der Analytaufnahmekammer eine vorgewählte Zeit vorliegt, und anschließend
Schritt b): gegebenenfalls analythaltiges Transportmedium aus der Analytaufnahmekammer in den Ableitungskanal transportiert wird. Eine solche Steuerung stimmt somit den Betrieb der ersten und zweiten Pumpe aufeinander ab, um das Durchtretenlassen eines Analyten in Transportmedium aus einer Probe zu gestatten und das gegebenenfalls analytbeladene Transportmedium aus der Analytaufnahmekammer herauszutransportieren. Zum Transportieren kann die Analytaufnahmekammer wie oben beschrieben mit Transportmedium, Spülmedium oder Kalibriermedium gespült werden.

Bevorzugt ist die Steuerung ferner eingerichtet, um die erste Pumpe so zu steuern, dass

Schritt c): nach Schritt b) gegebenenfalls analythaltiges Transportmedium aus dem Ableitungskanal zum Sensoranschluss transportiert wird.

Dieses Transportieren kann, muss aber nicht im Schritt 2 erfolgen. Wiederum kann in Schritt c) die Analytaufnahmekammer gespült werden mit Transportmedium, Kalibriermedium oder Spülmedium wie oben beschrieben. Wenn am Sennsoranschluss ein Sensor angeschlossen ist, dann ist die Steuerung zweckmäßigerweise so eingerichtet, dass das gegebenenfalls analythaltige Transportmedium in den Sensor transportiert wird.

Am Sensoranschluss eines erfindungsgemäßen Probenahmesystems ist vorzugsweise ein Sensor fluidleitend angebracht, der eingerichtet ist zum Bestimmen eines Analyten ausgewählt aus Glukose, Laktose, Laktat, Na⁺, K⁺, Cl⁻, H₃O⁺, O₂, CO₂, Ammonium, Ammoniak, Methanol, Ethanol, Formiat, Acetat, Glutamin, Glutamat, Harnstoff, Harnsäure, Phosphat, Antikörper, Wachstumsfaktoren, Hormone, Medikamente und/oder Narkotika.

Ein erfindungsgemäßes Probenahmeverfahren umfasst die Schritte:
1) Einsaugen einer Probe in eine Probenkammer einer erfindungsgemäßen Probenahmevorrichtung,
2) Bereitstellen eines Transportmediums in der Analytaufnahmekammer für eine vorgewählte Zeit zum Eintretenlassen eines gegebenenfalls in der Probe enthaltenen Analyten in die Analytaufnahmekammer,
3) Ausstoßen der in der Probenkammer eingesaugten Probe und
4) vor, gleichzeitig mit oder nach Schritt 3) Transportieren von Transportmedium aus der Analytaufnahmekammer. Ein erfindungsgemäßes Analyseverfahren entspricht dem erfindungsgemäßen Probenahmeverfahren, wobei im Schritt 4) das gegebenenfalls analytbeladene Transportmedium aus der Analytaufnahmekammer zu einem Sensor zum Bestimmen des Analyten geleitet wird. Das erfindungsgemäße Probenahmeverfahren und das erfindungsgemäße Analyseverfahren verwirklichen die oben beschriebenen Vorteile der Erfindung.

Die Analytaufnahmekammer und/oder der Sensor wird bzw. werden vorzugsweise in einem weiteren Schritt mit analytfreiem Transportmedium, Spülmedium oder Kalibriermedium gespült. Entsprechend eingerichtete Probenahmevorrichtungen und Probenahmesysteme erfindungsgemäßer Art sind oben beschrieben.

Bevorzugt ist daher ein solches erfindungsgemäßes Probenahmeverfahren bzw. erfindungsgemäßes Analyseverfahren, das ferner den Schritt umfasst des Beaufschlagens der Analytaufnahmekammer mit einem Kalibriermedium durch Zuführen des Kalibriermediums in die Analytaufnahmekammer durch den Zuleitungskanal.

Ebenfalls bevorzugt ist erfindungsgemäßes Probenahme- bzw. Analyseverfahren umfassend den Schritt:
Herstellen einer kalibrierten Konzentration eines Analyten in der Analytaufnahmekammer durch
   1. Zuführen des Kalibriermediums in die Probenkammer und
   2. während eines vorgewählten Zeitraums Durchtretenlassen von Analyt aus der Probenkammer in ein Transportmedium in der Analytaufnahmekammer.

Wenn ein Kalibriermedium in einem erfindungsgemäßen Probenahme- und/oder Analyseverfahren eingesetzt wird, dann ist es aus oben zur erfindungsgemäßen Probenahmevorrichtung und dem erfindungsgemäßen Probenahmesystem beschriebenen Gründen bevorzugt, dass Kalibriermedium durch einen Alternativauslass der Probenahmevorrichtung austreten zu lassen.

Die Erfindung wird nachfolgend anhand der Figuren und ausgewählter Ausführungsformen näher beschrieben, wobei diese Beschreibung den Schutzbereich der Patentansprüche nicht einschränkt.

Es stellen dar:
Fig. 1: schematische Querschnittdarstellung einer nicht erfindungsgemäßen Probenahmevorrichtung umfassend ein mit einem Trennmedium versehenes Fenster, durch das Probenkammer und Analytaufnahmekammer miteinander verbunden sind;
Fig. 2: schematische Querschnittdarstellung einer Probenahmevorrichtung gemäß Figur 1 mit Abstandhaltern;
Fig. 3: schematische Querschnittdarstellung einer Probenahmevorrichtung gemäß Figur 1 mit Filter,
Fig. 4: schematische Querschnittdarstellung einer erfindungsgemäßen Probenahmevorrichtung, wobei die Analytaufnahmekammer die Probenkammer umgibt und die Probenkammer als ein die Probenahmevorrichtung durchmessender Kanal ausgebildet ist;
Fig. 5: schematische Außenansicht einer Probenahmevorrichtung gemäß Figur 4, ausgestattet mit einem Luer-Anschluss;
Fig. 5a: schematische Ansicht einer weiteren Probenahmevorrichtung gemäß Figur 4 mit Luer-Anschluss;
Fig. 6a: schematische Außenansicht einer Probenahmevorrichtung, die in Form einer Sonde ausgelegt ist;

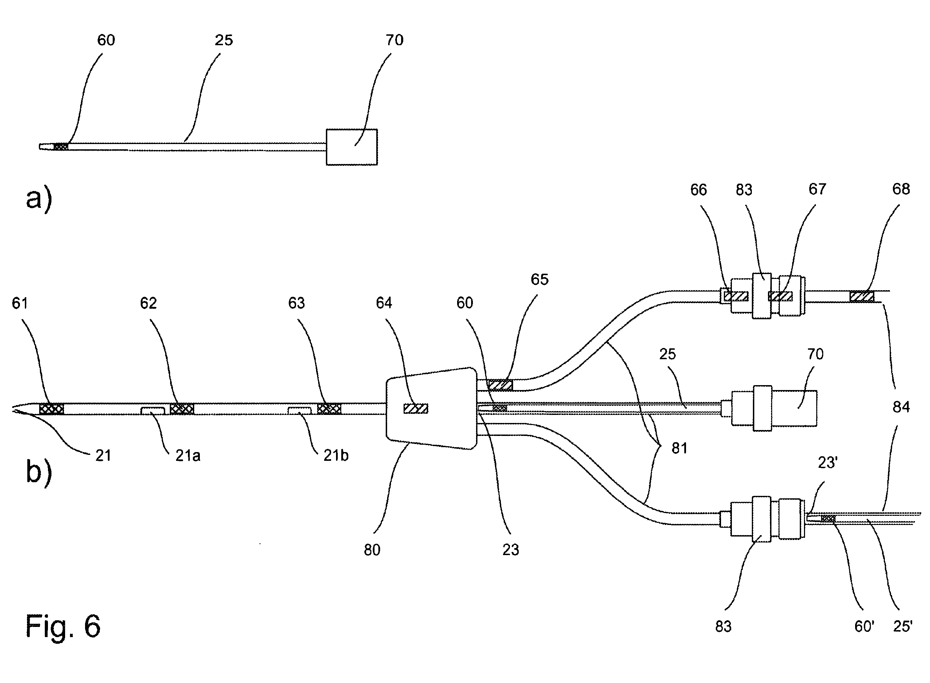

Fig. 6 b: schematische Darstellung der Anwendung von Probenahmevorrichtungen in Verbindung mit einem Probenahmezugang in Form eines Katheters;

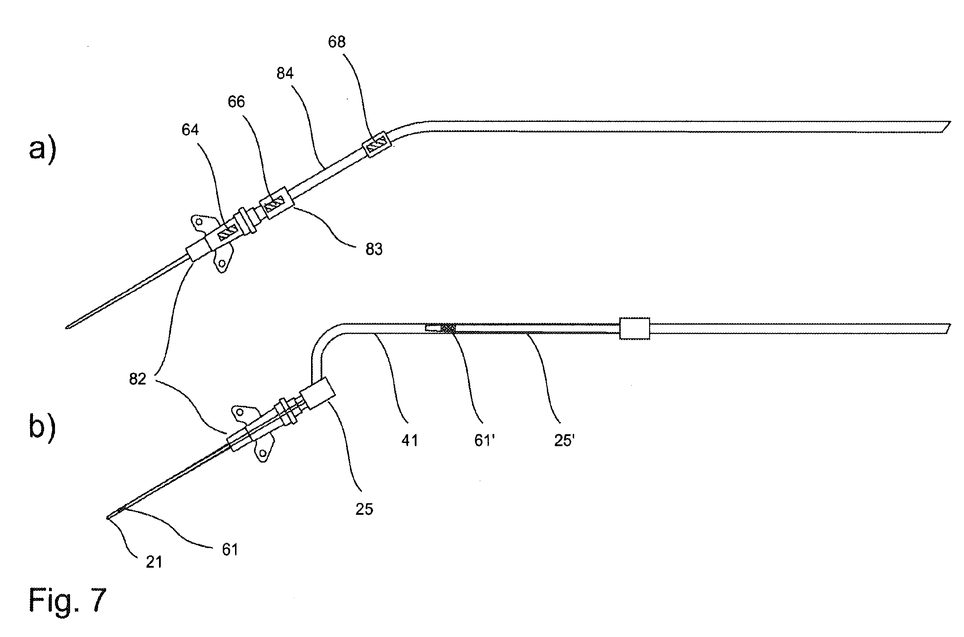

Fig. 7a, b: schematische Darstellung der Anwendung einer erfindungsgemäßen Probenahmevorrichtung in Verbindung mit einem Probenahmezugang in Form einer Kanüle;

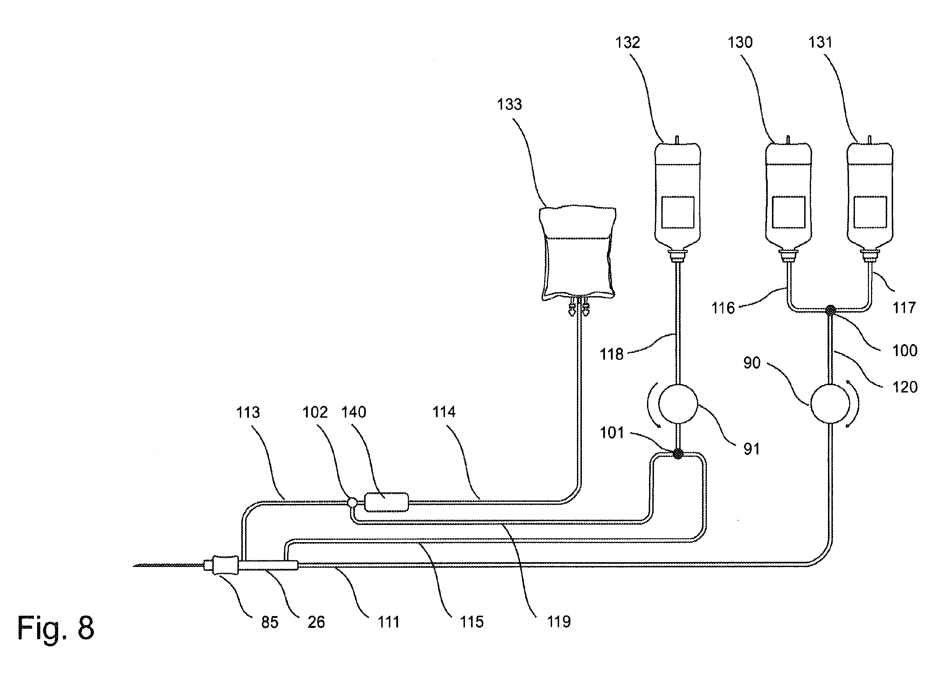

Fig. 8: schematische Darstellung eines erfindungsgemäßen Probenahmesystems;

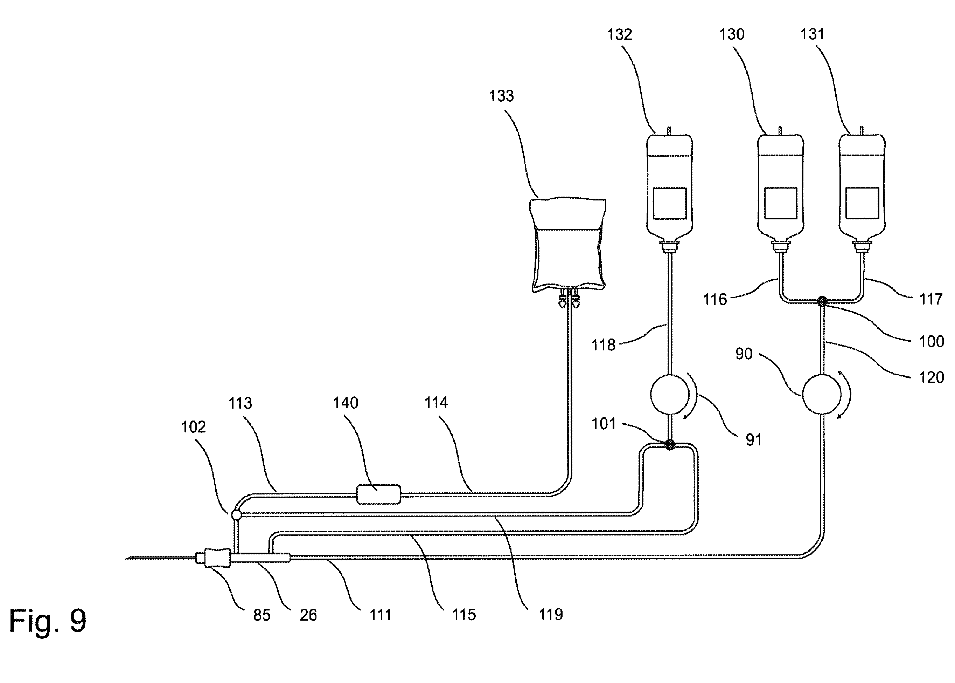

Fig. 9: schematische Darstellung einer Alternative zu dem erfindungsgemäßen Probenahmesystems gemäß Figur 8 für größere Entfernungen zwischen Probenahmevorrichtung und Sensor;

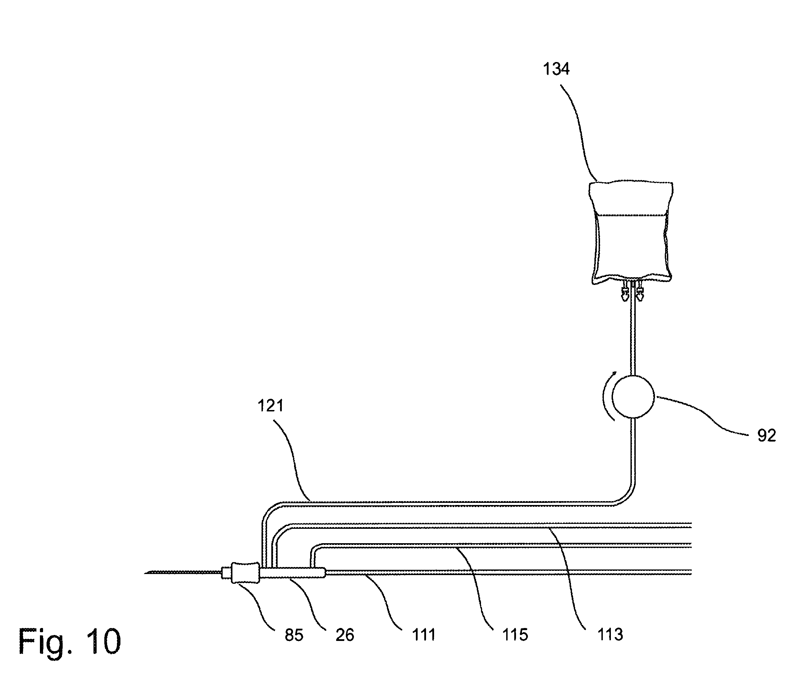

Fig. 10: schematische Darstellung einer weiteren Alternative zu dem erfindungsgemäßen Probenahmesystems gemäß Figur 8 mit einem Alternativauslass;

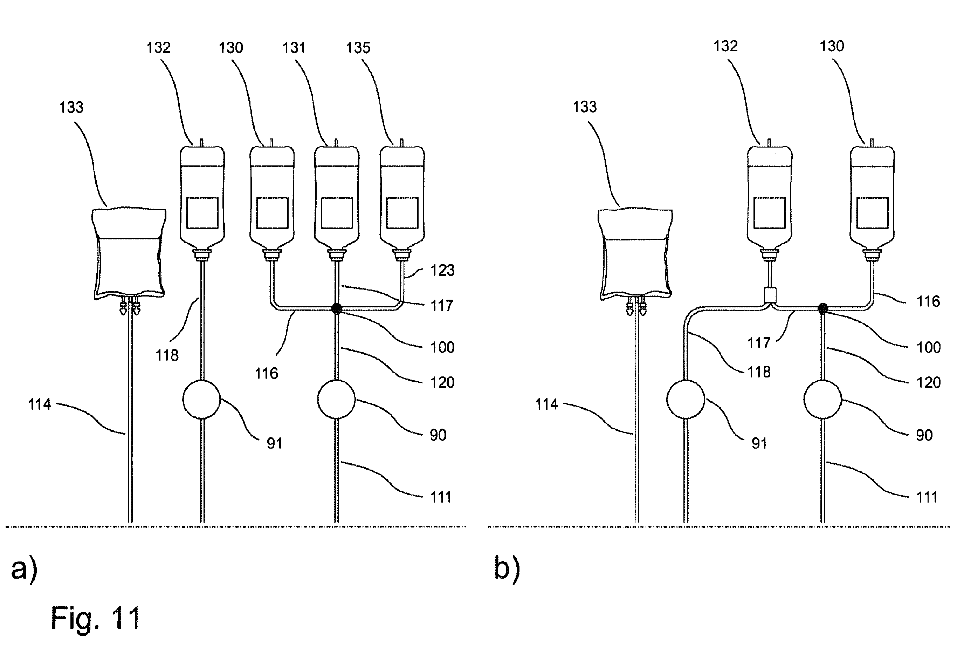

Fig. 11a, b: schematische Darstellung einer weiteren Alternative zu dem erfindungsgemäßen Probenahmesystems gemäß Figur 8 mit alternativen Fluidaufbewahrungs- und Zuleitungssystemen;

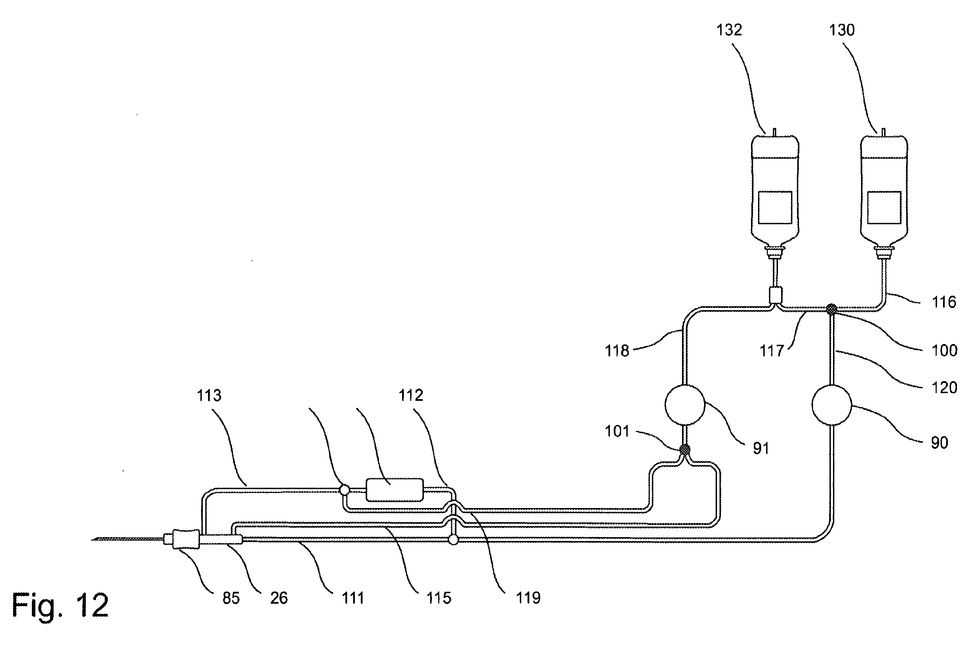

Fig. 12: schematische Darstellung einer weiteren sehr kompakten Alternative zu dem erfindungsgemäßen Probenahmesystems gemäß Figur 8 und

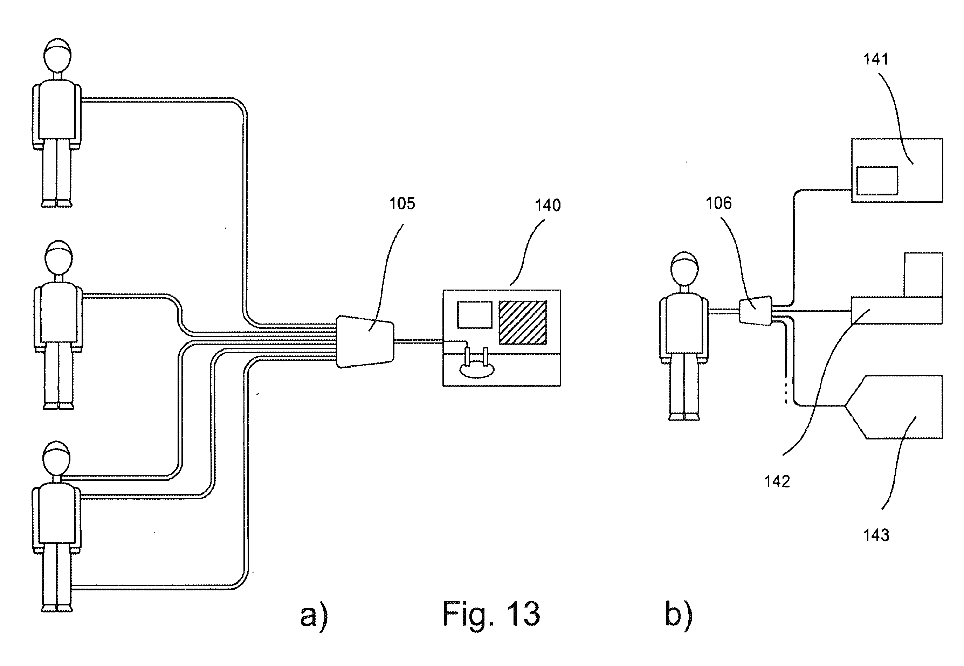

Fig. 13a, b schematische Darstellung von einer erfindungsgemäßen Probenahmevorrichtung mit mehreren Patienten oder mehreren Sensoren oder Messstationen

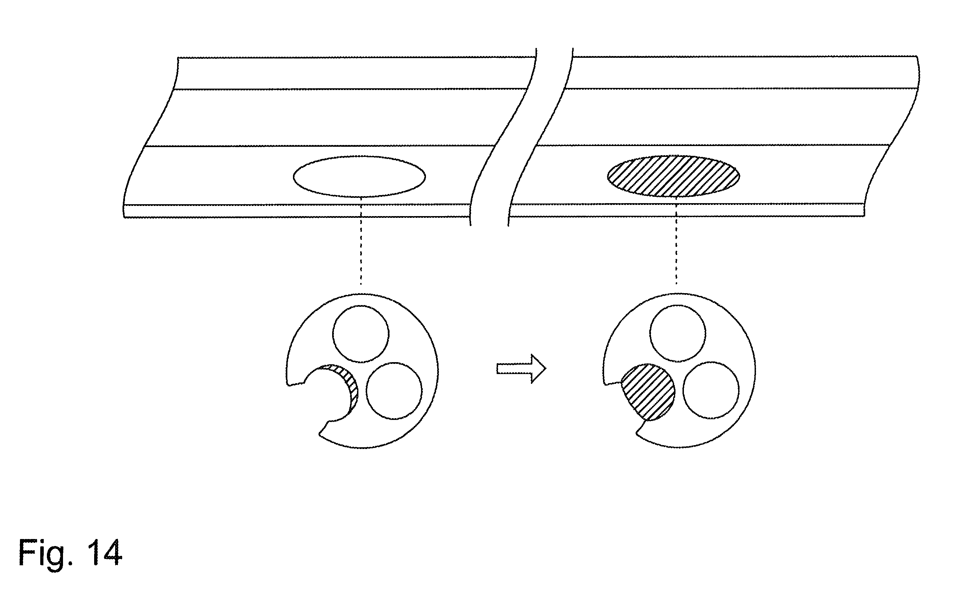

Fig 14: schematische Darstellung eines Katheters mit aufblähbarer Verschlussmembran.

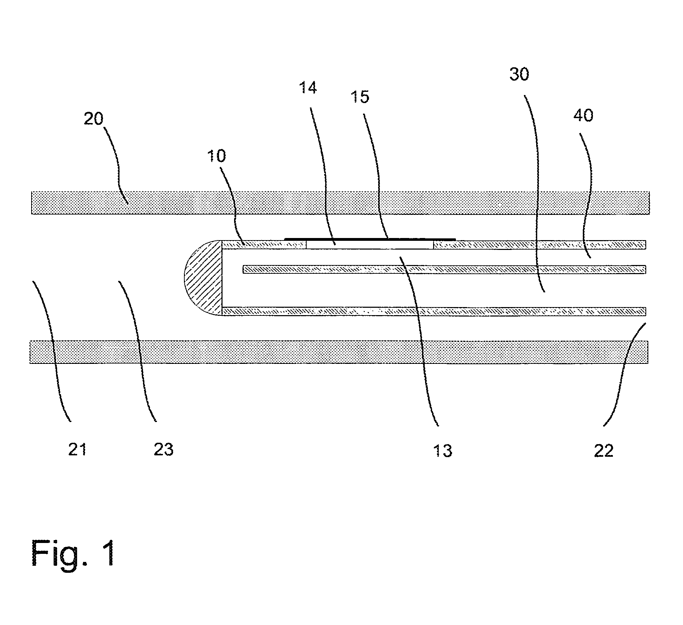

Figur 1 zeigt zu Vergleichszwecken eine nicht erfindungsgemäße Probenahmevorrichtung 10 mit einer Hohlnadel 20. Die Hohlnadel 20 bildet eine im Querschnitt kreisförmige Röhre, welche die Probenkammer 23 darstellt. Die Probenkammer 23 weist zwei Öffnungen auf, wobei die erste Öffnung der Probeneinlass 21 ist und angespitzt sein kann (nicht gezeigt), um das Einführen der Hohlnadel 20 in einen Patienten zu erleichtern. Die zweite Öffnung 22 ist durch eine Probenahmeleitung (nicht gezeigt) mit einer zweiten Pumpe 90 (nicht gezeigt) verbunden, um ein Spülmedium durch die Probenkammer 23 aus dem Probeneinlass 21 zum Spülen der Probenkammer 23 hinauszutreiben oder ein zu beprobendes Medium durch den Probeneinlass 21 in das Innere der Probenkammer 23 einzusaugen.

Die Probenahmevorrichtung 10 besitzt eine innere Ausnehmung 13. Die innere Ausnehmung 13 ist fluidleitend verbunden mit einem im Inneren der Probenahmevorrichtung 10 angeordneten Zuleitungskanal 30 und Ableitungskanal 40. Im Bereich der inneren Ausnehmung 13 ist die Außenwand der Probenahmevorrichtung 10 fensterförmig durchbro chen; das Fenster 14 ist durch eine Trennmembran 15 verschlossen. Aus der Probenkammer 23 kann ein Analyt durch die Trennmembran 15 in die innere Ausnehmung 13 hineindiffundieren; die innere Ausnehmung 13 bildet eine Analytaufnahmekammer.

Im Betrieb wird zunächst die Probenahmevorrichtung 10 in einen Patienten oder einen Probenahmezugang, beispielsweise eine Hohlnadel 20, insbesondere eine Kanüle, einen Katheter oder den Probenahmezugang eines Bioreaktors eingeführt. Handelt es sich bei der Hohlnadel 20 um eine 18 Gauge-Hohlnadel, so beträgt der Abstand des Probeneinlasses 21 von der Spitze der Probenahmevorrichtung 10 bevorzugt etwa 3 mm. Anschließend wird die Probenkammer 23 mit einem Spülmedium gespült. Gleichzeitig oder anschließend hieran wird die Analytaufnahmekammer 13 mit Transportmedium gespült.

In die gespülte Probenkammer 23 wird anschließend eine Probe eines zu beprobenden Mediums, beispielsweise Blut, eingesaugt, so dass die Trennmembran 15 im Bereich des Fensters 14 der Analytaufnahmekammer 10 vollständig oder in einem vorgewählten Maße unvollständig von der Probe bedeckt ist. Handelt es sich bei der Hohlnadel 20 um eine 18 Gauge-Hohlnadel, so reichen hierfür vorzugsweise 75 µl aus.

Nach dem Einsaugen der Probe wird eine vorgewählte Zeit gewartet, um den oder dem Analyten Gelegenheit zu geben, in die Analytaufnahmekammer 13 zu diffundieren. Alternativ dazu kann auch eine vorgewählte Zeit lang Probenmaterial in die Probenkammer 23 eingesaugt und optional ausgespült werden, um eine möglichst gleichbleibend hohe Analytkonzentration im Inneren der Probenkammer 23 sicherzustellen, so dass eine möglichst hohe Analytkonzentration in der Analytaufnahmekammer 13 erreicht werden kann.

Anschließend oder gleichzeitig wird durch Zuleitungskanal 30 Transportmedium gespült, um das Volumen des Transportmediums aus der Analytaufnahmekammer 13 durch Ableitungskanal 40 zu einem Sensor (nicht dargestellt) zu transportieren. Der Sensor ist eingerichtet zum qualitativen und/oder quantitativen Nachweisen des Analyten. Zweckmäßigerweise wird beim Spülen der Analytaufnahmekammer 13 auch das Innere der Probenkammer 23 mit einem Spülmedium gespült, um die Probe aus der Probenkammer 23 auszutreiben und weiteres Eindringen des Analyten in die Analytaufnahmekammer 13 zu begrenzen.

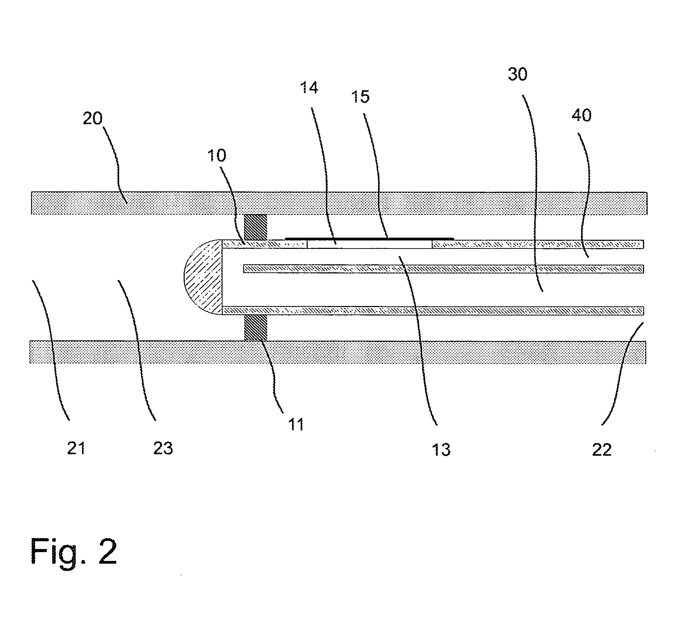

Figur 2 unterscheidet sich von Figur 1 lediglich dadurch, dass hier Abstandhalter 11 vorgesehen sind, um die Probenahmevorrichtung 10 von den Innenwänden der Hohlnadel 20 beabstandet zu halten. Auf diese Weise ist sichergestellt, dass die Trennmembran 15 im Bereich des Fensters 14 der Probenahmevorrichtung 10 für einen Übertritt des Analyten frei bleibt und nicht unbeabsichtigt ganz oder teilweise durch die Innenwand der Hohlnadel 20 verschlossen wird.

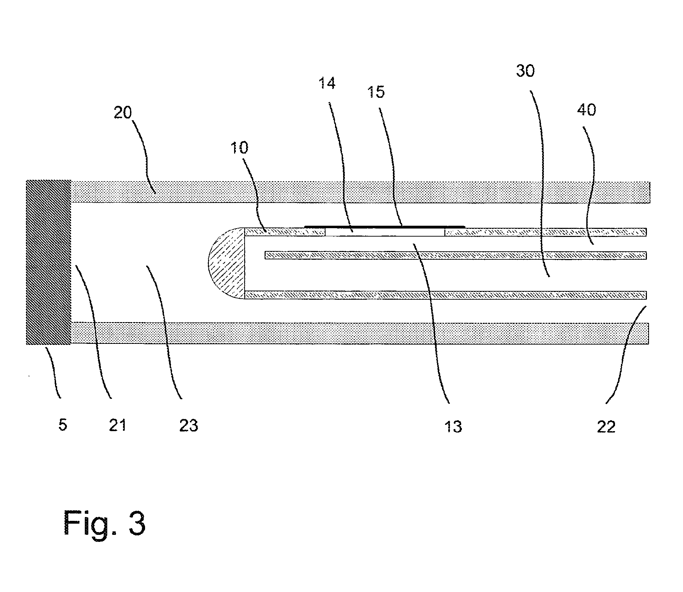

Figur 3 unterscheidet sich von Figur 1 lediglich darin, dass am Probeneinlass 21 ein Filter 5, vorzugsweise ein Sterilfilter, angebracht ist. So kann das Eintreten von Mikroorganismen in das Innere der Hohlnadel 20 verhindert werden. Dadurch kann der Bildung von Ablagerungen oder Biofilmen im Inneren der Hohlnadel 20 vorgebeugt werden. Außerdem ist es so möglich, Kontaminationen des zu beprobenden Mediums durch das Spülmedium zu verhindern. Insbesondere zum Einsatz an Bioreaktoren, bei denen das Austreten und Eindringen von Zellen in oder aus dem zu beprobenden Medium vermieden werden soll.

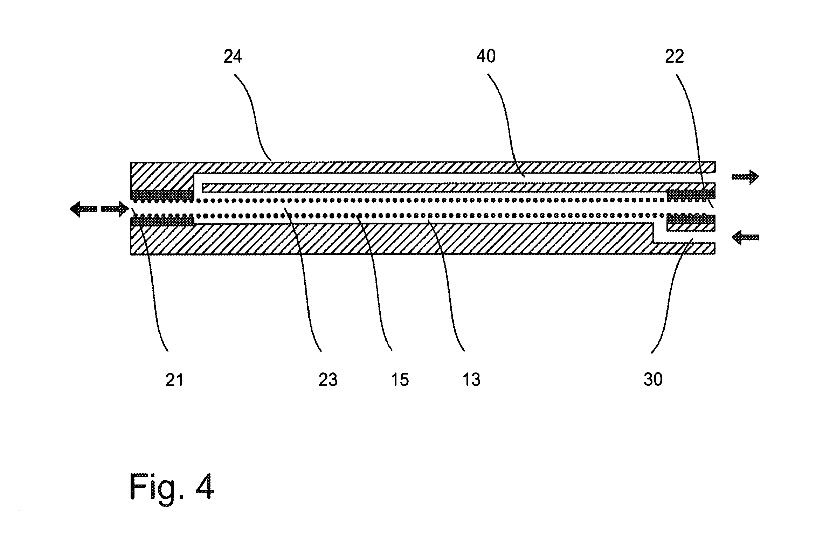

Figur 4 zeigt eine zylinderförmige Probenahmevorrichtung 24. Die Probenahmevorrichtung 24 umfasst eine Analytaufnahmekammer 13, in der sich eine ebenfalls zylinderförmige Probenkammer 23 befindet. Die Probenkammer 23 ist als ein die Probenahmevorrichtung 24 durchmessender Kanal ausgebildet und weist zwei Öffnungen auf, wobei die erste Öffnung der Probeneinlass 21 ist. Die zweite Öffnung 22 ist durch eine Probenahmeleitung (nicht gezeigt) mit einer zweiten Pumpe 90 (nicht gezeigt) verbunden, um ein Spülmedium durch die Probenkammer 23 aus dem Probeneinlass 21 zum Spülen der Probenkammer 23 hinauszutreiben oder ein zu beprobendes Medium durch den Probeneinlass 21 in das Innere der Probenkammer 23 einzusaugen.

Die Analytaufnahmekammer 13 ist mit einem Zuleitungskanal 30 und einem Ableitungskanal 40 versehen. Die Probenkammer 23 ist mit einer Trennmembran 15 versehen. Aus der Probenkammer 23 kann ein Analyt durch die Trennmembran 15 in die Analytaufnahmekammer 13 hineindiffundieren.

Im Betrieb wird in der entsprechenden Weise verfahren, wie dies für die Probenahmevorrichtung 10 gemäß Figur 1 vorstehend beschrieben wurde.

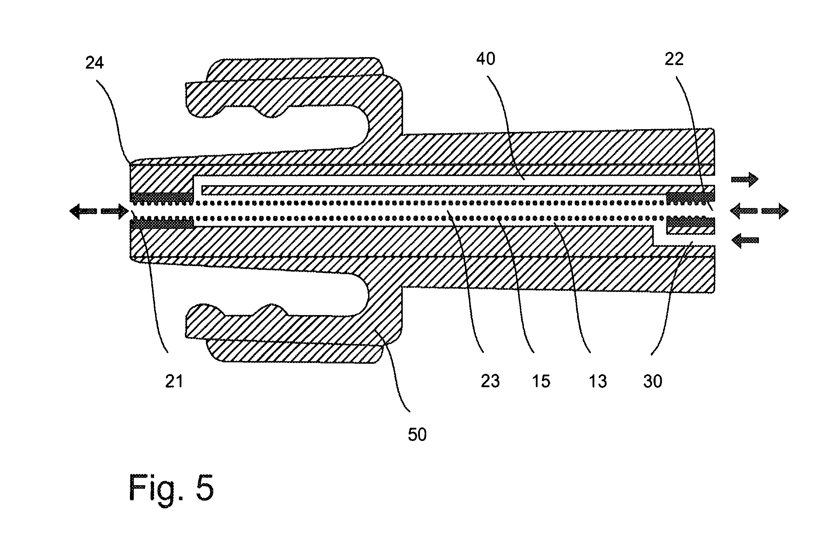

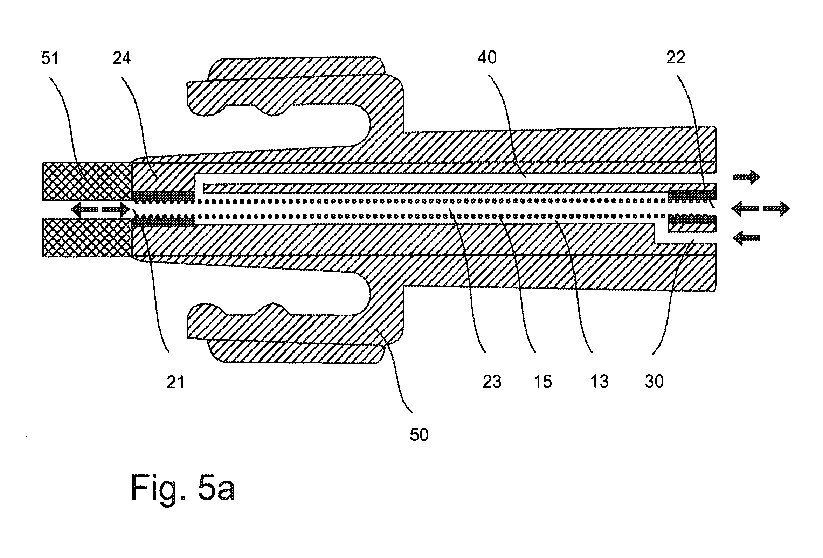

Figur 5 unterscheidet sich von Figur 4 lediglich dadurch, dass hier ein Luer-Anschluss 50 vorgesehen ist, mit dessen Hilfe die Probenahmevorrichtung 24 form-, kraft- und/oder reibschlüssig mit einem Probenahmezugang verbunden werden kann.

Figur 5a zeigt eine erfindungsgemäße Probenahmevorrichtung der in Figur 5 gezeigten Art, die zusätzlich einen Füllkörper 51 zum Verringern eines Totvolumens in einem Luer-Anschluss aufweist.

Figur 6a zeigt die schematische Außenansicht einer Probenahmevorrichtung 25, die in Form einer Sonde ausgelegt ist. Die Sonde besitzt eine Spitze, die nach Art der Probenahmevorrichtung 10 gemäß einer der Figuren 1 bis 3 ausgelegt ist. Die gekreuzte Schraffierung 60 zeigt die ungefähre Position der Trennmembran 15 der Probenahmevorrichtung 25 an. Zuleitungskanal 30 (nicht gezeigt) und Ableitungskanal 40 (nicht gezeigt) der Probenahmevorrichtung 25 können mit nicht dargestellten Fluidverbindern verbunden werden. Durch einen weiteren Fluidverbinder kann eine bei Einsatz der Probenahmevorrichtung 25 gebildete Probenkammer 23 (nicht gezeigt) mit einer Probenahmeleitung (nicht gezeigt) verbunden werden durch einen Anschluss 70.

Figur 6b zeigt verschiedene Möglichkeiten der Anwendung einer Probenahmevorrichtung, in Verbindung mit einem Probenahmezugang in Form eines Multilumenkatheters 80. Probenahmevorrichtung 25 ist außerhalb des Multilumenkatheters 80 an oder in einem Anschluss 81 angebracht, wobei sich sowohl die Probenkammern 23, 23' als auch die Trennmembran 15, deren Position durch die Schraffuren 60, 60' angezeigt wird, außerhalb des eigentlichen Katheters und außerhalb des Mediumhauptkörpers und bei Anwendung an Menschen auch außerhalb des menschlichen Körpers befindet. Schraffierungen 61, 62 und 63 zeigen weitere mögliche Positionen der Trennmembran 15 an, die erreicht werden, wenn Probenahmevorrichtung 25 tiefer in den Multilumenkatheter 80 eingebracht wird. Zu diesen Positionen zugeordnet sind die Probeneinlässe 21, 21a und 21b. Steht die Trennmembran 15 in der Position der Schraffierung 61, so steht der Probeneinlass 21 in direktem Kontakt mit dem Mediumhauptkörper.

Figur 6b zeigt ferner die Anwendung einer Probenahmevorrichtung 25' in einer Ab- oder Zuleitung des Multilumenkatheters 80. Die Probenahmevorrichtung 25' ist ausgestaltet wie die Probenahmevorrichtung 25.

Figur 6b zeigt außerdem durch die einfach schraffierten Flächen 64 bis 68 mögliche Positionen erfindungsgemäßer Probenahmevorrichtungen (nicht gezeigt, vergleiche Fig. 4 oder 5 als Beispiel) an, die mit dem Multilumenkatheter 80, seinen Zuleitungen 81, Fluidverbindern 83 oder mit externen Leitungen 84 einstückig verbunden sind.

Die Anordnung erlaubt also eine weitgehend freie Wahl des Ortes der Entnahme von Proben aus dem Mediumhauptkörper, dem Multilumenkatheter 80, deren Zuleitungen und/oder deren Fluidverbindern.

Figur 7 zeigt schematisch die Anwendung einer erfindungsgemäßen Probenahmevorrichtung in Verbindung mit einer Kanüle 82, zum Beispiel einer Venenverweilkanüle oder einer Arterienkanüle.

Figur 7a zeigt durch die einfach schraffierten Flächen 64, 66 und 68 mögliche Positionen erfindungsgemäßer Probenahmevorrichtungen (nicht gezeigt, vergleiche Fig. 4 oder 5 als Beispiel) an, die mit der Kanüle 82, Fluidverbindern 83 oder mit externen Leitungen 84 einstückig verbunden sind.

Figur 7b zeigt verschiedene Möglichkeiten der Anwendung der Probenahmevorrichtung 25, 25' in Verbindung mit einem Probenahmezugang in Form einer Kanüle 82. Probenahmevorrichtung 25 ist in die Kanüle 82 eingebracht. Die Schraffierung 61 zeigt die Position des Trennmediums 15 der Probenahmevorrichtung 25 an. Der Probeneinlass 21 steht bei dieser Anordnung in direktem Kontakt mit dem Mediumhauptkörper.

Probenahmevorrichtung 25', die einer Probenahmevorrichtung 25 entspricht, ist in eine mit der Kanüle 82 verbundenen Leitung 41 außerhalb des zu beprobenden Mediumhauptkörpers eingebracht. Die Schraffierung 61' zeigt die Position des Trennmediums 15 der Probenahmevorrichtung 25' an.

Figur 8 zeigt die schematische Darstellung eines erfindungsgemäßen Probenahmesystems. Probenahmezugang 85 ist mit Probenahmevorrichtung 26 versehen.

Das Probenahmesystem umfasst eine Probenfluidik, umfassend eine Probenahmeleitung 111, die mit der Probenkammer (nicht gezeigt) der Probenahmevorrichtung 26 verbunden ist und am anderen Ende mit Pumpe 90 ausgerüstet ist, die in beide Richtungen zu pumpen vermag. Die andere Seite der Pumpe ist durch Leitung 120 mit einem schaltbaren Anschluss 100 verbunden, dass außerdem durch Leitung 116 mit einem Behälter 130 für Kalibriermedium und durch Leitung 117 mit einem Behälter 131 für Spüllösung versehen ist. Schaltbarer Anschluss 100 kann so geschaltet werden, dass Leitung 116 oder Leitung 117 oder beide fluidleitend mit Leitung 120 verbunden sind.

Das Probenahmesystem umfasst weiter eine Analysefluidik, umfassend Zuleitung 115, die fluidleitend mit dem Zuleitungskanal (nicht gezeigt) der Probenahmevorrichtung 26 verbunden ist und am anderen Ende mit Pumpe 91 ausgerüstet ist. Die andere Seite der Pumpe ist durch Leitung 118 mit einem Behälter 132 für Transportmedium verbunden. Ferner umfasst die Analysefluidik des Probenahmesystems eine Ableitung 113, die fluidleitend mit dem Ableitungskanal (nicht gezeigt) der Probenahmevorrichtung 26 verbunden ist und außerdem mit Sensor 140 verbunden ist. Sensor 140 ist eingerichtet zum qualitativen und/oder quantitativen Nachweisen des Analyten. Sensor 140 ist außerdem über Leitung 114 mit Mediumabfall 133 verbunden. Zuleitung 115 ist außerdem mit dem schaltbaren Anschluss 101 und Ableitung 113 ist außerdem mit Verbinder 102 versehen. Schaltbarer Anschluss 101 und Verbinder 102 sind durch die Bypassleitung 119 verbunden.

Im Betrieb wird zunächst Probenahmezugang 85, beispielsweise eine Kanüle, ein Katheter oder der Probenahmezugang eines Bioreaktors mit dem Mediumhauptkörper in Berührung gebracht. Anschließend wird schaltbarer Anschluss 100 so eingestellt, dass Leitungen 117 und 120 fluidleitend verbunden sind und Pumpe 90 wird in Betrieb gesetzt und fördert die Spüllösung aus Behälter 131 durch Leitungen 117 und 120 und Probenahmeleitung 111 in die Probenkammer (nicht gezeigt) der Probenahmevorrichtung 26. Dabei wird zunächst der Inhalt der Probenkammer (nicht gezeigt) der Probenahmevorrichtung 26 und dann die Spüllösung in den Mediumhauptkörper eingeleitet (gegebenenfalls in den Patienten infundiert). Ist die Probenkammer (nicht gezeigt) der Probenahmevorrichtung 26 im Wesentlichen von Spüllösung ausgefüllt, wird Pumpe 90 gestoppt.

Gleichzeitig oder anschließend hieran wird die Analytaufnahmekammer (nicht gezeigt) der Probenahmevorrichtung 26 mit Transportmedium gespült, in dem Pumpe 91 eingeschaltet wird und schaltbarer Anschluss 101 so geschaltet wird, dass Pumpe 91 Transportmedium aus Behälter 132 durch Leitung 118, Zuleitung 115 in die Analytaufnahmekammer (nicht gezeigt) der Probenahmevorrichtung 26 und von dort weiter durch Ableitung 113, Sensor 140, Leitung 114 in den Mediumabfall 133 fördert. Hierbei sollte üblicherweise solange Transportmedium gefördert werden, bis das komplette Volumen der Analytaufnahmekammer (nicht gezeigt) der Probenahmevorrichtung 26 den Sensor 140 passiert hat und der Sensor 140 den Messwert für reines Transportmedium anzeigt. Anschließend kann die Pumpe 91 gestoppt und optional ein neuer Zyklus begonnen werden.

Durch Umkehr der Fließrichtung an Pumpe 90 und deren Inbetriebnahme wird die Spüllösung in Richtung der Pumpe 90 gefördert. Dabei strömt zu beprobendes Medium in die Probenkammer (nicht gezeigt) der Probenahmevorrichtung 26 ein, mischt sich dort mit der Spülflüssigkeit und wird dann zunächst im Gemisch mit der Spülflüssigkeit und bei andauernder Förderung später in reiner Form durch die Probenkammer (nicht gezeigt) der Probenahmevorrichtung 26 in die Probenahmeleitung 111 gefördert. Ist die Probenkammer (nicht gezeigt) der Probenahmevorrichtung 26 im Wesentlichen vollständig durch zu beprobendes Medium ausgefüllt, kann Pumpe 90 entweder gestoppt werden, wodurch das zu beprobende Medium in der Probenkammer (nicht gezeigt) der Probenahmevorrichtung 26 verweilt oder Alternativ dazu kann Pumpe 90 auch eine vorgewählte Zeit lang weiter betrieben und so Probenmaterial in die Probenkammer eingesaugt werden, um eine möglichst gleichbleibend hohe Analytkonzentration im Inneren der Probenkammer (nicht gezeigt) sicherzustellen, so dass eine möglichst hohe Konzentration des bzw. der Analyten in der Analytaufnahmekammer (nicht gezeigt) der Probenahmevorrichtung 26 erreicht werden kann. Für den letzteren Fall kann in der Probenahmeleitung 111 auch ein Auffangvolumen zur Zwischenlagerung des zu beprobenden Mediums vorgesehen werden. Anstatt der Spüllösung kann auch die Kalibrierlösung aus Behälter 130 durch entsprechende Einstellung des schaltbaren Anschlusses 100 für diesen Prozess verwendet werden. Die Kalibrierlösung kann auch kontinuierlich in den Mediumhauptkörper eingeleitet (im Falle eines Patienten infudiert) werden.

Ist die Probenkammer (nicht gezeigt) der Probenahmevorrichtung 26 im Wesentlichen mit zu beprobendem Medium ausgefüllt, so wird eine vorgewählte Zeit gewartet, um dem Analyten Gelegenheit zu geben, in die Analytaufnahmekammer 13 (nicht gezeigt) zu diffundieren. Während der optionalen Wartezeit kann das Transportmedium aus Behälter 132 entweder direkt durch Bypass 119 zum Sensor 140 geleitet werden oder Pumpe 91 kann abgestellt werden. Ebenfalls möglich ist es, in die Probenkammer ständig weiteres Medium einzusaugen, und erst nach Abfuhr des gegebenenfalls analytbeladenen Transportmediums zum Sensor das Medium wieder auszustoßen. Und ferner ist es möglich, das eingesaugte Medium wie eingangs beschrieben in einer Schlaufe zurückzuführen.

Durch eine geeignete Abfolge lässt sich mit dieser Methode auch ein zu beprobendes Medium in Kontakt mit der Trennschicht (nicht gezeigt) der Probenahmevorrichtung 26 bringen, während gleichzeitig Anreicherung und Abtransport auf der anderen Seite der Trennschicht (nicht gezeigt) der Probenahmevorrichtung 26 stattfinden können. Selbstverständlich lassen sich die Fluidiken auf beiden Seiten auch kontinuierlich pulsierend oder alternierend betreiben.

Figur 9 zeigt eine schematische Darstellung einer alternativen Form des Probenahmesystems gemäß Figur 8. Hierbei ist Verbinder 102 sehr nahe an der Probenahmevorrichtung 26 angebracht oder einstückig damit verbunden. Dadurch kann der Sensor in einer größeren Distanz zur Probenahmevorrichtung 26 angebracht werden, Dieser Aufbau empfiehlt sich zum Beispiel, wenn größere Distanzen zwischen Probenahmevorrichtung 26 und Sensor 140 vorliegen oder wenn mehrere Probenahmevorrichtungen 26 an einen Sensor 140 angeschlossen werden.

Figur 10 zeigt eine schematische Darstellung einer alternativen Form des Probenahmesystems gemäß Figur 8. Hierbei ist Probenahmevorrichtung 26 zusätzlich mit einem Alternativauslass 121 versehen, der mit der Probenkammer (nicht gezeigt) der Probenahmevorrichtung 26 im Bereich zwischen dem Probeneinlass (nicht gezeigt) und dem Trennmedium (nicht gezeigt) der Probenahmevorrichtung 26 fluidleitend verbunden ist. Alternativauslass 121 ist mit Pumpe 92 und weiter mit Abfallbehälter 134 verbunden.

Dies ermöglicht es, die Probenkammer (nicht gezeigt) der Probenahmevorrichtung 26 mit Kalibriermedium und/oder Spülmedium und/oder Transportmedium zu spülen und das zum Spülen verwendete Medium statt in den Vorrat des zu beprobenden Mediums durch den Alternativauslass abzupumpen. Hierdurch kann zum Beispiel die Volumenbelastung (mit Flüssigkeiten) eines Patienten reduziert werden.

Figur 11 zeigt schematische Darstellungen eines Ausschnitts des Probenahmesystems gemäß Figur 8 mit alternativen Fluidaufbewahrungs- und Zuleitungssystemen.

Figur 11 a zeigt den Aufbau der Fluidaufbewahrungs- und Fluidzuleitungssystemen bei Einsatz eines zusätzlichen Behälters 135 für eine zusätzliche Kalibrierlösung. Die Behälter 130 und 135 mit den Kalibrierlösungen und der Behälter 131 mit der Spüllösung sind dabei über die Leitungen 116, 117 und 123 mit dem schaltbaren Anschluss 100 verbunden, dass wiederum über Leitung 120 mit Pumpe 90 verbunden ist. Durch schaltbaren Anschluss 100 lässt sich je einer oder weitere der Behälter 130, 131 und 135 fluidleitend mit Leitung 120 verbinden. Dieser Aufbau eignet sich für den Einsatz von mehreren Kalibrierlösungen die zum Beispiel verschiedene Konzentrationen des Analyten enthalten und damit für Sensoren, die mehrere Kalibrierungspunkte benötigen.

Figur 11b zeigt den Aufbau der Fluidaufbewahrungs- und Fluidzuleitungssysteme bei dem die Leitungen für das Spül- und das Transportmittel zusammengeführt werden können. Dies ist ein besonders platzsparendes System, dass ein kompaktes Probenahmesystem mit wenigen Medienbehältern erlaubt. Dabei bildet das Transportmedium auch das Spülmedium, so dass die Probenahmeleitung 111 und die Zuleitung entweder wie dargestellt über einen schaltbaren Anschluss 100 mit dem Vorrat an Transportmedium und Spülmedium angeschlossen ist, oder an einem gemeinsamen Vorratsbehälter angeschlossen ist (nicht dargestellt).

Figur 12 zeigt die schematische Darstellung einer weiteren sehr kompakten Alternative zu dem erfindungsgemäßen Probenahmesystems gemäß Figur 8. Hierbei ist das Fluidaufbewahrungs- und Fluidzuleitungssystem wie in Figur 11b aufgebaut. Zusätzlich ist jedoch der Behälter 133 für den Mediumabfall weggelassen worden. Das aus dem Sensor 140 nach der Messung austretende Medium wird hier mittels Leitung 112 in die Probenahmeleitung 111 eingeleitet. Dies stellt eine kompakte Anordnung dar. Hierbei müssen jedoch das gesamte Schlauchsystem, der Analyseanteil und die Medien steril ausgeführt sein. Bevorzugt wird ein komplett geschlossenes steriles Fluid-System beispielsweise in Kassettenformat vorkonfektioniert bereitgestellt. Der gesamte Ablauf muss so eingestellt werden, dass das analytbeladene Segment herausgespült wird ohne die darauffolgende Messung zu verfälschen.

Figur 13 zeigt eine schematische Darstellung einer erfindungsgemäßen Probenahmevorrichtung mit mehreren Patienten und einem Sensor und eine Messanordnung mit einem Patienten und mehreren Sensoren. Es handelt sich dabei prinzipiell um dieselbe Probenahmevorrichtung, die auch in Figur 8 gezeigt ist. Figur 13a zeigt, dass mehrere Probenahmevorrichtungen 26 (nicht gezeigt) in mehreren Patienten und/oder auch mehrere Probenahmevorrichtungen 26 (nicht gezeigt) in einem Patient über einen schaltbaren Anschluss 105 mit einem einzigen Sensor 140 verbunden sein können. Dies bietet sich zum Beispiel an, wenn ein besonders aufwendiges oder kompliziertes Analyseverfahren angewendet wird, dass sich jedoch automatisieren lässt. Figur 13b zeigt, dass eine Probenahmevorrichtung 26 (nicht gezeigt) in einem Patient über einen schaltbaren Anschluss 106 mit mehreren Sensoren 141, 142 und 143 verbunden sein kann. Dies bietet sich zum Beispiel an, wenn verschiedene Analyte bestimmt werden müssen.

Figur 14 zeigt schematisch einen 3-Lumen-Katheter mit einem Katheterfenster zum Eintretenlassen von zu beprobendem Medium in einen Innenkanal des Katheters. Der Innenkanal weist eine bewegliche Verschlussmembran auf, die durch Beaufschlagen mit einem Medium (rechts dargestellt) zum ballonartigen Aufblähen der Verschlussmembran (schraffiert) und somit zu einer Volumenverringerung des Innenkanals führt. In der dargestellten Ausführungsform ist die Verschlussmembran so bemessen, dass die aufgeblähte Verschlussmembran das Katheterfenster gegen den Durchtritt eines Mediums verschließt (rechts oben dargestellt).

Weitere erfindungsgemäße und nicht-erfindungsgemäße Ausführungsformen werden nachfolgend beschrieben:
1. Probenahmevorrichtung für einen Probenahmezugang, umfassend
   - eine Analytaufnahmekammer zum Aufnehmen eines Analyten in einem Transportmedium, verbunden mit einem Zuleitungskanal und einem Ableitungskanal zum Transportieren von Transportmedium in die bzw. von der Analytaufnahmekammer,
   - eine als innere Ausnehmung ausgebildete Probenkammer mit einem Probeneinlass zum Aufnehmen einer gegebenenfalls analythaltigen Probe, und
   - eine die innere Ausnehmung umgebendes Trennmedium zum Durchtretenlassen des Analyten aus der Probenkammer in die Analytaufnahmekammer.
2. Probenahmevorrichtung gemäß Ausführungsform 1, dadurch gekennzeichnet, dass die Analytaufnahmekammer die Probenkammer umgibt.
3. Probenahmevorrichtung gemäß einer der vorherigen Ausführungsformen, dadurch gekennzeichnet, dass die Probenkammer als ein die Probenahmevorrichtung durchmessender Kanal ausgebildet ist.
4. Probenahmevorrichtung gemäß einer der vorherigen Ausführungsformen, dadurch gekennzeichnet, dass die Probenahmevorrichtung ausgestaltet ist zum Anbringen an einem medizinischen Probenahmezugang, vorzugsweise einer Hohlnadel (Kanüle), einem Katheter, einem Port, einem Sauger, einer Drainage, einem Reservoir und/oder einem Konnektor.
5. Probenahmevorrichtung gemäß einer der vorherigen Ausführungsformen, dadurch gekennzeichnet, dass die Probenahmevorrichtung ausgestaltet ist zum form- und/oder kraft- und/oder reibschlüssigen Verbinden mit einem Probenahmezugang, vorzugsweise mit einem Luer-Anschluss und/oder einem Schraubverschluss.
6. Probenahmevorrichtung gemäß einer der vorherigen Ausführungsformen, dadurch gekennzeichnet, dass die Probenahmevorrichtung ausgestaltet ist zum Anordnen der Analytaufnahmekammer
   - im Inneren eines zu beprobenden Mediumhauptkörpers durch einen Probenahmezugang hindurch,
   - in einem Probenahmezugang,
   - an einem bezogen auf einen zu beprobenden Mediumhauptkörper abgewandten Ende eines Probenahmezugangs, und/oder
   - in einer mit einem Probenahmezugang verbundenen Leitung außerhalb eines zu beprobenden Mediumhauptkörpers.
7. Probenahmevorrichtung gemäß einer der vorherigen Ausführungsformen 2 bis 4, dadurch gekennzeichnet, dass die Probenahmevorrichtung mit dem Probenahmezugang einstückig verbunden ist.
8. Probenahmevorrichtung gemäß einer der vorherigen Ausführungsformen, dadurch gekennzeichnet, dass die Probenahmevorrichtung ausgestaltet ist zum Aufnehmen einer Probe von Körperflüssigkeit, vorzugsweise ausgewählt aus Blut, Blutplasma, Lymphe, Gewebeflüssigkeit, Liquor cerebrospinalis, Synovia, Magensaft, Galle und Urin.
9. Probenahmevorrichtung gemäß einer der vorherigen Ausführungsformen, dadurch gekennzeichnet, dass das Trennmedium eine Membran ist.
10. Probenahmevorrichtung gemäß Ausführungsform 9, dadurch gekennzeichnet, dass das Material der Probenmembran ausgewählt ist aus der Gruppe bestehend aus Cellulose und deren Derivaten, insbesondere Celluloseacetat, PTFE, Polycarbonat, Polypropylen, Polyamiden, Polyestern, Polyestersulfonen und Polysulfonen.
11. Probenahmevorrichtung gemäß einer der vorherigen Ausführungsformen, dadurch gekennzeichnet, dass das Trennmedium ausgestaltet ist zum Durchtretenlassen eines Analyten ausgewählt aus Glucose, Lactose, Lactat, Na⁺, K⁺, Cl⁻, H₃O⁺, O₂, CO₂, Ammonium, Ammoniak, Methanol, Ethanol, Formiat, Acetat, Glutamin, Glutamat, Harnstoff, Harnsäure, Phosphat, Antikörper, Wachstumsfaktoren, Hormonen, Medikamenten und Narkotika.
12. Probenahmevorrichtung gemäß einer der vorherigen Ausführungsformen, dadurch gekennzeichnet, dass der Zuleitungskanal und/oder der Ableitungskanal an der Analytaufnahmekammer einen Innendurchmesser von 0,2 - 0,3 mm besitzt bzw. besitzen, vorzugsweise von 0,2 - 0,3 mm, besonders bevorzugt 0,23 - 0,26 mm.
13. Probenahmevorrichtung gemäß einer der vorherigen Ausführungsformen, dadurch gekennzeichnet, dass die Fläche desjenigen Trennmediumabschnittes, der die Probenkammer mit der Analytaufnahmekammer verbindet (Membranfenster), 0,5 - 350 mm² beträgt, vorzugsweise 1 - 50 mm² und besonders bevorzugt 2 - 35 mm².
14. Probenahmevorrichtung gemäß Ausführungsform 13, dadurch gekennzeichnet, dass das Verhältnis der Fläche des Membranfensters zum Innendurchmesser des Ableitungskanals größer als 400 ist.
15. Probenahmevorrichtung gemäß einer der vorherigen Ausführungsformen, dadurch gekennzeichnet, dass das Volumen der Analytaufnahmekammer 2-50 mm³ beträgt.
16. Probenahmevorrichtung gemäß einer der vorherigen Ausführungsformen, ferner umfassend eine bewegliche Verschlussmembran zum Verringern und/oder Verschließen eines Volumens der Probenkammer oder des Probenahmezuganges.
17. Probenahmevorrichtung gemäß Ausführungsform 16, ferner umfassend einen Verschluss-Steuerungskanal zum Ausüben eines Druckes zum Bewegen der Verschlussmembran.
18. Probenahmevorrichtung gemäß einer der vorherigen Ausführungsformen, dadurch gekennzeichnet, dass die Probenkammer ferner einen Alternativauslass umfasst zum Austretenlassen von in der Probenkammer aufgenommenem Medium.
19. Probenahmesystem, umfassend
   - eine Probenahmevorrichtung, vorzugsweise gemäß einer der vorherigen Ausführungsformen,
   - einen Sensoranschluss zum Anschließen eines Sensors zum Nachweisen eines Analyten in einem Transportmedium,
   - eine Zuleitung, fluidleitend verbunden mit dem Zuleitungskanal der Probenahmevorrichtung,
   - eine Ableitung, fluidleitend verbunden mit dem Ableitungskanal der Probenahmevorrichtung und dem Sensoranschluss,
   - eine erste Pumpe zum Transportieren eines Mediums durch die Ableitung zum Sensoranschluss.
20. Probenahmesystem gemäß Ausführungsform 19, ferner umfassend eine Probenahmeleitung und mit dieser verbunden eine zweite Pumpe zum Pumpen einer Probe in die Probenkammer.
21. Probenahmesystem gemäß einer der Ausführungsformen 19 bis 20, ferner umfassend:
   - einen Transportmediumvorrat und/oder
   - einen Kalibriermediumvorrat und/oder
   - einen Spülmediumvorrat, der bzw. die mit der Zuleitung fluidleitend verbunden oder verbindbar ist bzw. sind.
22. Probenahmesystem gemäß einer der vorherigen Ausführungsformen 19 bis 21, dadurch gekennzeichnet, dass die erste Pumpe angeordnet ist zwischen a) dem Transportmediumvorrat und/oder dem Kalibriermediumvorrat und/oder dem Spülmediumvorrat und b) dem Zuleitungskanal der Probenahmevorrichtung zum Transportieren von Medium vom jeweiligen Mediumvorrat durch die Zuleitung in den Zuleitungskanal.
23. Probenahmesystem gemäß einer der vorherigen Ausführungsformen 19 bis 22, ferner umfassend eine Bypassleitung zum fluidleitenden Verbinden des Transportmediumvorrats und/oder des Kalibriermediumvorrats und/oder des Spülmediumvorrats mit dem Sensoranschluss ohne Passieren der Analytaufnahmekammer der Probenahmevorrichtung.
24. Probenahmesystem gemäß Ausführungsform 23, ferner umfassend einen schaltbaren Anschluss zum fluidleitenden Verbinden der Bypassleitung mit der Ableitung.
25. Probenahmesystem gemäß einer der vorherigen Ausführungsformen 23 bis 24, ferner umfassend einen schaltbaren Anschluss zum alternativen fluidleitenden Verbinden des Transportmediumvorrats und/oder des Kalibriermediumvorrats und/oder des Spülmediumvorrats mit entweder der Zuleitung oder der Bypassleitung.
26. Probenahmesystem gemäß einer der vorherigen Ausführungsformen 19 bis 25, ferner umfassend eine Ausflussleitung zum fluidleitenden Verbinden der Ableitung über den Sensoranschluss und gegebenenfalls einen Sensor mit der Probenahmeleitung.
27. Probenahmesystem gemäß Ausführungsform 26, ferner umfassend einen schaltbaren Anschluss zum alternativen fluidleitenden Verbinden
   a) der Ableitung mit der Ausflussleitung gegebenenfalls über einen Sensor, oder
   b) der Ableitung mit einer Abfallleitung gegebenenfalls über einen Sensor zum Leiten von aus der Ableitung gegebenenfalls durch den Sensor austretendem Medium zu einer Mediumentsorgung.
28. Probenahmesystem gemäß einer der vorherigen Ausführungsformen 19 bis 27, ferner umfassend einen schaltbaren Anschluss zum Verbinden des Transportmediumvorrats und/oder des Kalibriermediumvorrats und/oder des Spülmediumvorrats mit der Probenahmeleitung über die zweite Pumpe.
29. Probenahmesystem gemäß einer der vorherigen Ausführungsformen 19 bis 28, ferner umfassend eine Steuerung, verbunden mit der zweiten Pumpe, um die zweite Pumpe so zu steuern, dass
   Schritt 1: eine Probe in die Probenkammer eingesaugt wird,
   Schritt 2: die eingesaugte Probe eine vorgewählte Zeit in der Probenkammer vorliegt, und anschließend
   Schritt 3: die eingesaugte Probe aus der Probenkammer ausgestoßen wird.
30. Probennahmesystem gemäß Ausführungsform 29, ferner umfassend eine Hohlnadel, wobei die Analytaufnahmekammer in der Hohlnadel angeordnet ist.
31. Probenahmesystem gemäß einer der vorherigen Ausführungsformen 29 bis 30, dadurch gekennzeichnet, dass die Steuerung ferner verbunden ist mit der ersten Pumpe, um diese so zu steuern, dass in Schritt 2:
   Schritt a: Transportmedium in der Analytaufnahmekammer eine vorgewählte Zeit vorliegt, und anschließend
   Schritt b: ggf. analythaltiges Transportmedium aus der Analytaufnahmekammer in den Ableitungskanal transportiert wird.
32. Probenahmesystem gemäß einer der vorherigen Ausführungsformen 29 bis 31, dadurch gekennzeichnet, dass die Steuerung ferner eingerichtet ist, um die erste Pumpe so zu steuern, dass
   Schritt c: nach Schritt b) ggf. analythaltiges Transportmedium aus dem Ableitungskanal zum Sensoranschluss transportiert wird.
33. Probenahmesystem, umfassend
   - eine Hohlnadel,
   - eine Probenahmevorrichtung mit einer Analytaufnahmekammer in fluidleitender Verbindung zwischen einer Zuleitung und einer Ableitung, wobei die Analytaufnahmekammer eine Öffnung besitzt, die verschlossen ist mit einer analytpermeablen Membran zum Durchtretenlassen des Analyten von einem Bereich außerhalb der Analytaufnahmekammer in die Analytaufnahmekammer, und wobei die Analytaufnahmekammer in der Hohlnadel angeordnet ist,
   - eine erste Pumpvorrichtung zum Transportieren eines Transportmediums durch die Analytaufnahmekammer,
   - eine zweite Pumpvorrichtung zum Ansaugen und optional Ausstoßen eines zu analysierenden Mediums durch die Hohlnadel, sowie
   - eine Steuerung in Wirkverbindung mit der ersten Pumpvorrichtung, wobei die Steuerung eingerichtet ist, das Transportmedium zu einem vorgewählten Zeitpunkt durch die Analytaufnahmekammer zum Gewinnen einer Probe des gegebenenfalls analythaltigen Transportmediums pumpen zu lassen.
34. Probenahmesystem gemäß einer der vorherigen Ausführungsformen 19 bis 33, ferner umfassend in fluidleitender Verbindung mit dem Sensoranschluss einen Sensor eingerichtet zum Bestimmen eines Analyten ausgewählt aus Glucose, Lactose, Lactat, Na⁺, K⁺, Cl⁻, H₃O⁺, O₂, CO₂, Ammonium, Ammoniak, Methanol, Ethanol, Formiat, Acetat, Glutamin, Glutamat, Harnstoff, Harnsäure, Phosphat, Antikörper, Wachstumsfaktoren, Hormonen, Medikamenten und Narkotika.
35. Probenahmeverfahren, umfassend die Schritte:
   1) Einsaugen einer Probe in eine Probenkammer einer Probenahmevorrichtung gemäß einer der Ausführungsformen 19 bis 34, wobei gegebenenfalls das Innere der Hohlnadel eines Probenahmesystems gemäß Ausführungsform 33 die Probenkammer bildet,
   2) Bereitstellen eines Transportmediums in der Analytaufnahmekammer für eine vorgewählte Zeit zum Eintretenlassen eines ggf. in der Probe enthaltenen Analyten in die Analytaufnahmekammer,
   3) Ausstoßen der in die Probenkammer eingesaugten Probe und
   4) vor, gleichzeitig mit oder nach Schritt 3) Transportieren von Transportmedium aus der Analytaufnahmekammer.
36. Probenahmeverfahren gemäß Ausführungsform 35, ferner umfassend den Schritt: Spülen der Analytaufnahmekammer und/oder des Sensors mit analytfreiem Transportmedium, Spülmedium oder Kalibriermedium.
37. Probenahmeverfahren gemäß einer der vorherigen Ausführungsformen 35 bis 36, ferner umfassend den Schritt: Beaufschlagen der Analytaufnahmekammer mit einem Kalibriermedium durch Zuführen des Kalibriermediums in die Analytaufnahmekammer durch den Zuleitungskanal.
38. Probenahmeverfahren gemäß einer der vorherigen Ausführungsformen 35 bis 37, ferner umfassend den Schritt: Herstellen einer kalibrierten Konzentration eines Analyten in der Analytaufnahmekammer durch
   1. Zuführen des Kalibriermediums in die Probenkammer und
   2. während eines vorgewählten Zeitraums Durchtretenlassen von Analyt aus der Probenkammer in ein Transportmedium in der Analytaufnahmekammer.
39. Probenahmeverfahren gemäß Ausführungsform 38, ferner umfassend den Schritt:
   - Austretenlassen des Kalibriermediums durch einen Alternativauslass der Probenahmevorrichtung.
40. Analyseverfahren, umfassend die Schritte: Durchführen eines Probenahmeverfahren gemäß einer der vorherigen Ausführungsformen 35 bis 39, wobei in Schritt 4) das gegebenenfalls analythaltige Transportmedium zu einem Sensor zum Bestimmen des Analyten transportiert wird.

## Patentansprüche

1. Probenahmevorrichtung (10) für einen Probenahmezugang, umfassend
- eine Analytaufnahmekammer (13) zum Aufnehmen eines Analyten in einem Transportmedium, verbunden mit einem Zuleitungskanal (30) und einem Ableitungskanal (40) zum Transportieren von Transportmedium in die bzw. von der Analytaufnahmekammer,
- eine als innere Ausnehmung ausgebildete Probenkammer (23) mit einem Probeneinlass (21) zum Aufnehmen einer gegebenenfalls analythaltigen Probe, und
- eine die innere Ausnehmung umgebendes Trennmedium (15) zum Durchtretenlassen des Analyten aus der Probenkammer in die Analytaufnahmekammer.

2. Probenahmevorrichtung gemäß Anspruch 1, **dadurch gekennzeichnet, dass** die Analytaufnahmekammer die Probenkammer umgibt.

3. Probenahmevorrichtung gemäß einem der Ansprüche 1 bis 2, **dadurch gekennzeichnet,**
- **dass** die Probenkammer als ein die Probenahmevorrichtung durchmessender Kanal ausgebildet ist, und/oder
- **dass** die Probenahmevorrichtung ausgestaltet ist zum Anbringen an einem medizinischen Probenahmezugang, vorzugsweise einer Hohlnadel (Kanüle), einem Katheter, einem Port, einem Sauger, einer Drainage, einem Reservoir und/oder einem Konnektor, und/oder
- **dass** die Probenahmevorrichtung ausgestaltet ist zum form- und/oder kraft- und/oder reibschlüssigen Verbinden mit einem Probenahmezugang, vorzugsweise mit einem Luer-Anschluss und/oder einem Schraubverschluss.

4. Probenahmevorrichtung nach einem der Ansprüche 1 bis 2, **dadurch gekennzeichnet, dass** die Probenahmevorrichtung mit dem Probenahmezugang einstückig verbunden ist, wobei der Probenahmezugang vorzugsweise ein medizinischer Probenahmezugang ist, besonders bevorzugt eine Hohlnadel (Kanüle), ein Katheter, ein Port, ein Sauger, eine Drainage, ein Reservoir und/oder ein Konnektor.

5. Probenahmevorrichtung nach einem der Ansprüche 1, 2, 3 oder 4, **dadurch gekennzeichnet, dass**
- die Probenahmevorrichtung eingerichtet ist zum Aufnehmen einer Probe von Körperflüssigkeit, vorzugsweise ausgewählt aus Blut, Blutplasma, Lymphe, Gewebeflüssigkeit, Liquor cerebrospinalis, Synovia, Magensaft, Galle und Urin, und/oder
- das Trennmedium ausgestaltet ist zum Durchtretenlassen eines Analyten ausgewählt aus Glukose, Laktose, Laktat, Na⁺, K⁺, Cl-, H₃O⁺, O₂, CO₂, Ammonium, Ammoniak, Methanol, Ethanol, Formiat, Acetat, Glutamin, Glutamat, Harnstoff, Harnsäure, Phosphat, Antikörpern, Wachstumsfaktoren, Hormonen, Medikamenten und insbesondere Narkotika.

6. Probenahmevorrichtung nach einem der Ansprüche 1, 2, 3, 4 oder 5, **dadurch gekennzeichnet, dass** Trennmedium eine Membran ist, und vorzugsweise das Material der Membran ausgewählt aus der Gruppe bestehend aus Zellulose und deren Derivaten, insbesondere Zelluloseacetat, PTFE, Polykarbonat, Polypropylen, Polyamiden, Polyestern, Polyethersulfonen und Polysulfonen.

7. Probenahmevorrichtung nach einem der Ansprüche 1, 2, 3, 4, 5 oder 6, **dadurch gekennzeichnet, dass**
- der Zuleitungskanal und/oder der Ableitungskanal an der Analytaufnahmekammer einen Innendurchmesser von -0,2 - 0,3 mm besitzt bzw. besitzen, vorzugsweise von 0,2 - 0,3 mm, besonders bevorzugt 0,23 - 0,26 mm, und/oder
- die Fläche desjenigen Trennmediumabschnittes, der die Probenkammer mit der Analytaufnahmekammer verbindet (Membranfenster), 0,5 - 350 mm² beträgt, vorzugsweise 1 - 50 mm² und besonders bevorzugt 2 - 35 mm².

8. Probenahmevorrichtung nach einem der Ansprüche 1, 2, 3, 4, 5, 6 oder 7, **dadurch gekennzeichnet, dass**
- die Probenkammer ferner einen Alternativauslass umfasst zum Austretenlassen von in der Probenkammer aufgenommenem Medium, und/oder
- dass die Probenahmevorrichtung eine bewegliche Verschlussmembran zum Verringern und/oder Verschließen eines Probenvolumens der Probenkammer und /oder des Probenahmezugangs besitzt.

9. Probenahmesystem, umfassend
- eine Probenahmevorrichtung nach einem der vorherigen Ansprüche,
- einen Sensoranschluss zum Anschließen eines Sensors zum Nachweisen eines Analyten in einem Transportmedium,
- eine Zuleitung, fluidleitend verbunden mit dem Zuleitungskanal der Probenahmevorrichtung,
- eine Ableitung, fluidleitend verbunden mit dem Ableitungskanal der Probenahmevorrichtung und dem Sensoranschluss,
- eine erste Pumpe zum Transportieren eines Mediums durch die Ableitung zum Sensoranschluss, und optional
- eine Probenahmeleitung und mit dieser verbunden eine zweite Pumpe zum Pumpen einer Probe in die Probenkammer.

10. Probenahmesystem nach Anspruch 9, ferner umfassend:
- einen Transportmediumvorrat und/oder
- einen Kalibriermediumvorrat und/oder
- einen Spülmediumvorrat,
der bzw. die mit der Zuleitung fluidleitend verbunden oder verbindbar ist bzw. sind.

11. Probenahmesystem nach Anspruch 10, wobei die erste Pumpe angeordnet zwischen a) dem Transportmediumvorrat und/oder dem Kalibriermediumvorrat und/oder dem Spülmediumvorrat und b) dem Zuleitungskanal der Probenahmevorrichtung zum Transportieren von Medium vom jeweiligen Mediumvorrat durch die Zuleitung in den Zuleitungskanal, und vorzugsweise ferner umfassend eine Steuerung verbunden mit der ersten Pumpe, um diese so zu steuern, dass
Schritt a: Transportmedium in der Analytaufnahmekammer eine vorgewählte Zeit vorliegt, und anschließend
Schritt b: ggf. analythaltiges Transportmedium aus der Analytaufnahmekammer in den Ableitungskanal transportiert wird.

12. Probenahmesystem nach einem der Ansprüche 9, 10 oder 11, ferner umfassend eine Steuerung, verbunden mit der zweiten Pumpe, um die zweite Pumpe so zu steuern, dass
Schritt 1: eine Probe in die Probenkammer eingesaugt wird,
Schritt 2: die eingesaugte Probe eine vorgewählte Zeit in der Probenkammer vorliegt, und anschließend
Schritt 3: die eingesaugte Probe aus der Probenkammer ausgestoßen wird,
wobei die Steuerung vorzugsweise so eingerichtet ist, dass die erste Pumpe so gesteuert wird, dass in Schritt 2
Schritt a: Transportmedium in der Analytaufnahmekammer eine vorgewählte Zeit vorliegt, und anschließend
Schritt b: ggf. analythaltiges Transportmedium aus Analytaufnahmekammer in den Ableitungskanal transportiert wird.

13. Probenahmesystem nach einem der Ansprüche 9, 10, 11 oder 12, ferner umfassend
in fluidleitender Verbindung mit dem Sensoranschluss einen Sensor eingerichtet zum Bestimmen eines Analyten ausgewählt Glukose, Laktose, Laktat, Na⁺, K⁺, Cl⁻, H₃O⁺, O₂, CO₂, Ammonium, Ammoniak, Methanol, Ethanol, Formiat, Acetat, Glutamin, Glutamat, Harnstoff, Harnsäure, Phosphat, Antikörpern, Wachstumsfaktoren, Hormonen, Medikamenten und insbesondere Narkotika, und/oder
wobei das Probenahmesystem eingerichtet ist zum Aufnehmen einer Probe von Körperflüssigkeit, vorzugsweise ausgewählt aus Blut, Blutplasma, Lymphe, Gewebeflüssigkeit, Liquor cerebrospinalis, Synovia, Magensaft, Galle und Urin, und/oder
wobei das Probenahmesystem ferner eine Bypassleitung umfasst zum fluidleitenden Verbinden des allfällig vorhandenen Transportmediumvorrats und/oder des Kalibriermediumvorrats und/oder des Spülmediumvorrats mit dem Sensoranschluss ohne Passieren der Analytaufnahmekammer der Probenahmevorrichtung.

14. Probenahmeverfahren, umfassend die Schritte:
1) Einsaugen einer Probe in eine Probenkammer einer Probenahmevorrichtung gemäß einem der Ansprüche 9, 10, 11, 12 oder 13,
2) Bereitstellen eines Transportmediums in der Analytaufnahmekammer für eine vorgewählte Zeit zum Eintretenlassen eines ggf. in der Probe enthaltenen Analyten in die Analytaufnahmekammer,
3) Ausstoßen der in die Probenkammer eingesaugten Probe und
4) vor, gleichzeitig mit oder nach Schritt 3) Transportieren von Transportmedium aus der Analytaufnahmekammer.

## Claims

1. Sampling device (10) for a sampling access, comprising
- an analyte receiving chamber (13) for receiving an analyte in a transporting medium, connected to an infeed-line passage (30) and an outfeed-line passage (40) for transporting transporting medium respectively into and out of the analyte receiving chamber,
- a sample chamber (23) taking the form of an internal cavity, having a sample inlet (21) for receiving a sample possibly containing analyte, and
- a separating medium (15) surrounding the internal cavity, to allow the analyte to pass through from the sample chamber into the analyte receiving chamber.

2. Sampling device according to Claim 1, **characterised in that** the analyte receiving chamber surrounds the sample chamber.

3. Sampling device according to one of Claims 1 to 2, **characterised**
- **in that** the sample chamber takes the form of a passage extending through the sampling device, and/or
- **in that** the sampling device is configured for fitting to a medical sampling access, preferably a hollow needle (cannula), a catheter, a port, an aspirator, a drain, a reservoir and/or a connector, and/or
- **in that** the sampling device is configured for connection by positive interengagement, force and/or friction to a sampling access, preferably to a Luer lock connector and/or a screwed connector.

4. Sampling device according to one of Claims 1 to 2, **characterised in that** the sampling device is integrally connected to the sampling access, the sampling access preferably being a medical sampling access, particularly preferably a hollow needle (cannula), a catheter, a port, an aspirator, a drain, a reservoir and/or a connector.

5. Sampling device according to one of Claims 1, 2, 3 or 4, **characterised in that**
- the sampling device is configured to receive a sample of body fluid, preferably selected from blood, blood plasma, lymph, tissue fluid, cerebrospinal fluid, synovial fluid, gastric juice, gall and urine, and/or
- the separating medium is configured to allow the passage of an analyte selected from glucose, lactose, lactate, Na⁺, K⁺, Cl⁻, H₃O⁺, O₂, CO₂, ammonium, ammonia, methanol, ethanol, formate, acetate, glutamine, glutamate, urea, uric acid, phosphate, antibodies, growth factors, hormones, medicaments, and in particular narcotics.

6. Sampling device according to one of Claims 1, 2, 3, 4 or 5, **characterised in that** the separating medium is a membrane, and the material of the membrane is preferably selected from the group consisting of cellulose and derivatives thereof, in particular cellulose acetate, PTFE, polycarbonate, polypropylene, polyamides, polyesters, polyethersulphones and polysulphones.

7. Sampling device according to one of Claims 1, 2, 3, 4, 5 or 6, **characterised in that**
- the inside diameter of the infeed-line passage and/or the outfeed-line passage, at the analyte receiving chamber, is from -0.2 - 0.3 mm, preferably from 0.2 - 0.3 mm, particularly preferably 0.23 - 0.26 mm, and/or
- the area of that portion of the separating medium that connects the sample chamber to the analyte receiving chamber (the membrane window) is 0.5 - 350 mm², preferably 1 - 50 mm² and particularly preferably 2 - 35 mm².

8. Sampling device according to one of Claims 1, 2, 3, 4, 5, 6 or 7, **characterised in that**
- the sample chamber further comprises an alternative outlet to allow medium received in the sample chamber to leave, and/or
- **in that** the sampling device has a movable closing-off membrane for reducing and/or closing off a sample volume of the sample chamber and/or of the sampling access.

9. Sampling system, comprising
- a sampling device according to one of the preceding claims,
- a sensor connection for connecting a sensor for detecting an analyte in a transporting medium,
- an infeed line, in fluid communication with the infeed-line passage of the sampling device,
- an outfeed line, in fluid communication with the outfeed-line passage of the sampling device and with the sensor connection,
- a first pump for transporting a medium through the outfeed line to the sensor connection and, as an option,
- a sampling line and, connected thereto, a second pump for pumping a sample into the sample chamber.

10. Sampling system according to Claim 9, further comprising:
- a supply of transporting medium and/or
- a supply of calibrating medium and/or
- a supply of flushing medium,
that are/is in fluid communication, or can be placed in fluid communication, with the infeed line.

11. Sampling system according to Claim 10, wherein the first pump is arranged between a) the supply of transporting medium and/or the supply of calibrating medium and/or the supply of flushing medium and b) the infeed-line passage of the sampling device, for transporting medium from the respective supply of medium through the infeed line into the infeed-line passage, and preferably further comprising a control system connected to the first pump, to control the latter in such a way that
Step a: transporting medium is present in the analyte receiving chamber for a preselected time and then
Step b: transporting medium possibly containing analyte is transported from the analyte receiving chamber into the outfeed-line passage.

12. Sampling system according to one of Claims 9, 10 or 11, further comprising a control system, connected to the second pump, to control the second pump in such a way that
Step 1: a sample is drawn by suction into the sample chamber,
Step 2: the sample that was drawn in is present in the sample chamber for a preselected time and then
Step 3: the sample that was drawn in is expelled from the sample chamber,
the control system preferably being so configured that the first pump is controlled in such a way that, in step 2
Step a: transporting medium is present in the analyte receiving chamber for a preselected time and then
Step b: transporting medium possibly containing analyte is transported from the analyte receiving chamber into the outfeed-line passage.

13. Sampling system according to one of Claims 9, 10, 11 or 12, further comprising,
in fluid communication with the sensor connection, a sensor configured to determine an analyte selected from glucose, lactose, lactate, Na⁺, K⁺, Cl⁻, H₃O⁺, O₂, CO₂, ammonium, ammonia, methanol, ethanol, formate, acetate, glutamine, glutamate, urea, uric acid, phosphate, antibodies, growth factors, hormones, medicaments, and in particular narcotics, and/or
the sampling system being configured to receive a sample of body fluid, preferably selected from blood, blood plasma, lymph, tissue fluid, cerebrospinal fluid, synovial fluid, gastric juice, gall and urine, and/or
the sampling system further comprising a bypass line for the fluid communication of the supply of transporting medium and/or the supply of calibrating medium and/or the supply of flushing medium, that may be present, with the sensor connection, without the medium passing through the analyte receiving chamber of the sampling device.

14. Sampling method, comprising the steps:
1) drawing of a sample by suction into a sample chamber of a sampling device according to one of Claims 9, 10, 11, 12 or 13,
2) making available of a transporting medium in the analyte receiving chamber for a preselected time to allow an analyte possibly contained in the sample to enter the analyte receiving chamber,
3) expulsion of the sample that was drawn into the sample chamber, and
4) before, simultaneously with, or after step 3), transport of transporting medium out of the analyte receiving chamber.

## Revendications

1. Dispositif d'échantillonnage (10) pour une voie d'échantillonnage, comprenant
- une chambre de réception d'analyte (13) pour recevoir un analyte dans un milieu de transport, reliée à un canal d'amenée (30) et un canal d'évacuation (40) pour transporter le milieu de transport dans, respectivement depuis, la chambre de réception d'analyte,
- une chambre à échantillons (23) en forme d'évidement interne avec un orifice d'entrée d'échantillons (21) pour recevoir un échantillon contenant éventuellement un analyte, et
- un milieu de séparation (15) entourant l'évidement interne pour laisser passer l'analyte de la chambre à échantillons à la chambre de réception d'analyte.

2. Dispositif d'échantillonnage selon la revendication 1, **caractérisé en ce que** la chambre de réception d'analyte entoure la chambre à échantillons.

3. Dispositif d'échantillonnage selon l'une des revendications 1 à 2, **caractérisé en ce que**
- la chambre à échantillons est en forme de canal traversant le dispositif d'échantillonnage, et/ou
- le dispositif d'échantillonnage est conçu pour le montage sur une voie d'échantillonnage médicale, de préférence une aiguille creuse (canule), un cathéter, un port, un dispositif d'aspiration, un drainage, un réservoir et/ou un connecteur, et/ou
- le dispositif d'échantillonnage est conçu pour le raccordement à engagement de forme et/ou à force et/ou par friction avec une voie d'échantillonnage, de préférence avec un raccord Luer et/ou un raccord à vis.

4. Dispositif d'échantillonnage selon l'une des revendications 1 à 2, **caractérisé en ce que** le dispositif d'échantillonnage est raccordé en un bloc à la voie d'échantillonnage, la voie d'échantillonnage étant de préférence une voie d'échantillonnage médicale, particulièrement préférentiellement une aiguille creuse (canule), un cathéter, un port, un dispositif d'aspiration, un drainage, un réservoir et/ou un connecteur.

5. Dispositif d'échantillonnage selon l'une des revendications 1, 2, 3, ou 4, **caractérisé en ce que**
- le dispositif d'échantillonnage est aménagé pour recevoir un échantillon de fluide corporel, de préférence choisi parmi le sang, le plasma sanguin, la lymphe, le liquide interstitiel, le liquide cérébrospinal, la synovie, le suc gastrique, la bile et l'urine, et/ou
- le milieu de séparation est conçu pour laisser passer un analyte choisi parmi le glucose, le lactose, le lactate, le Na⁺, le K⁺, le Cl⁻, le H₃O⁺, le O₂, le CO₂, l'ammonium, l'ammoniac, le méthanol, l'éthanol, le formiate, l'acétate, la glutamine, le glutamate, l'urée, l'acide urique, le phosphate, les anticorps, les facteurs de croissance, les hormones, les médicaments et en particulier les narcotiques.

6. Dispositif d'échantillonnage selon l'une des revendications 1, 2, 3, 4 ou 5, **caractérisé en ce que** le milieu de séparation est une membrane, et de préférence la matière de la membrane est choisie dans le groupe constitué par la cellulose et ses dérivés, en particulier l'acétate de cellulose, le PTFE, le polycarbonate, le polypropylène, les polyamides, les polyesters, les polyéther-sulfones, et les polysulfones.

7. Dispositif d'échantillonnage selon l'une des revendications 1, 2, 3, 4, 5 ou 6, **caractérisé en ce que**
- le canal d'amenée et/ou le canal d'évacuation sur la chambre de réception d'analyte présente(nt) un diamètre interne de -0,2 - 0,3 mm, de préférence de 0,2 - 0,3 mm, particulièrement préférentiellement de 0,23 - 0,26 mm, et/ou
- la surface de cette portion du milieu de séparation, qui relie la chambre à échantillons à la chambre de réception d'analyte (fenêtre membranaire), est de 0,5 - 350 mm², de préférence de 1 - 50 mm² et particulièrement préférentiellement de 2 - 35 mm².

8. Dispositif d'échantillonnage selon l'une des revendications 1, 2, 3, 4, 5, 6 ou 7, **caractérisé en ce que**
- la chambre à échantillons comprend en outre un orifice de sortie alternatif pour laisser sortir le milieu reçu dans la chambre à échantillons, et/ou
- le dispositif d'échantillonnage possède une membrane de fermeture mobile pour réduire et/ou obturer un volume d'échantillons de la chambre à échantillons et/ou de la voie d'échantillonnage.

9. Système d'échantillonnage, comprenant
- un dispositif d'échantillonnage selon l'une des revendications précédentes,
- un raccord de capteur pour raccorder un capteur pour la détection d'un analyte dans un milieu de transport,
- une amenée, reliée en communication de fluide au canal d'amenée du dispositif d'échantillonnage,
- une évacuation, reliée en communication de fluide au canal d'évacuation du dispositif d'échantillonnage et au raccord de capteur,
- une première pompe pour transporter un milieu à travers l'évacuation jusqu'au raccord de capteur, et éventuellement
- une conduite d'échantillonnage et, raccordée à celle-ci, une deuxième pompe pour pomper un échantillon dans la chambre à échantillons.

10. Système d'échantillonnage selon la revendication 9, comprenant en outre :
- une réserve de milieu de transport et/ou
- une réserve de milieu de calibrage et/ou
- une réserve de milieu de rinçage,
qui sont reliées ou peuvent être reliées en communication de fluide à l'amenée.

11. Système d'échantillonnage selon la revendication 10, dans lequel la première pompe est disposée entre a) la réserve de milieu de transport et/ou la réserve de milieu de calibrage et/ou la réserve de milieu de rinçage et b) le canal d'amenée du dispositif d'échantillonnage pour le transport du milieu depuis la réserve de milieu en question au canal d'amenée en passant par l'amenée, et de préférence comprenant en outre une commande raccordée à la première pompe pour commander celle-ci de manière que
étape a : le milieu de transport soit présent dans la chambre de réception de l'analyte pendant un temps présélectionné et ensuite
étape b : le milieu de transport contenant le cas échéant l'analyte soit transporté de la chambre de réception de l'analyte au canal d'évacuation.

12. Système d'échantillonnage selon l'une des revendications 9, 10, ou 11, comprenant en outre une commande, raccordée à la deuxième pompe pour commander la deuxième pompe de manière que
étape 1 : un échantillon soit aspiré dans la chambre à échantillons,
étape 2 : l'échantillon aspiré soit présent dans la chambre à échantillons pendant un temps présélectionné, et ensuite
étape 3 : l'échantillon aspiré soit expulsé de la chambre à échantillons,
la commande étant de préférence conçue de sorte que la première pompe soit commandée pour que, à l'étape 2
étape a : le milieu de transport soit présent dans la chambre de réception de l'analyte pendant un temps présélectionné et ensuite
étape b : le milieu de transport contenant le cas échéant l'analyte soit transporté de la chambre de réception de l'analyte au canal d'évacuation.

13. Système d'échantillonnage selon l'une des revendications 9, 10, 11 ou 12, comprenant en outre
un capteur, en communication de fluide avec le raccord de capteur, conçu pour la détermination d'un analyte choisi parmi le glucose, le lactose, le lactate, le Na⁺, le K⁺, le Cl⁻, le H₃O⁺, le O₂, le CO₂, l'ammonium, l'ammoniac, le méthanol, l'éthanol, le formiate, l'acétate, la glutamine, le glutamate, l'urée, l'acide urique, le phosphate, les anticorps, les facteurs de croissance, les hormones, les médicaments et en particulier les narcotiques, et/ou
le dispositif d'échantillonnage étant aménagé pour recevoir un échantillon de fluide corporel, de préférence choisi parmi le sang, le plasma sanguin, la lymphe, le liquide interstitiel, le liquide cérébrospinal, la synovie, le suc gastrique, la bile et l'urine, et/ou
le système d'échantillonnage comprenant en outre une conduite de contournement pour raccorder en communication de fluide la réserve de milieu de transport éventuellement présente et/ou la réserve de milieu de calibrage et/ou la réserve de milieu de rinçage au raccord de capteur sans passer par la chambre de réception de l'analyte du dispositif d'échantillonnage.

14. Procédé d'échantillonnage comprenant les étapes suivantes :
1) l'aspiration d'un échantillon dans une chambre à échantillons d'un dispositif d'échantillonnage selon l'une des revendications 9, 10, 11, 12 ou 13,
2) la mise à disposition d'un milieu de transport dans la chambre de réception d'analyte pour un temps présélectionné afin de laisser entrer, dans la chambre de réception d'analyte, un analyte contenu le cas échéant dans l'échantillon,
3) l'expulsion de l'échantillon aspiré dans la chambre à échantillons et
4) le transport du milieu de transport depuis la chambre de réception d'analyte avant, pendant ou après l'étape 3).
